# EUROPEAN PATENT APPLICATION

(11) **EP 1 138 317 A2**
(43) Date of publication of application: **04.10.2001**
(21) Application number: 01108164.3
(22) Date of filing: 30.03.2001
(51) Int. Cl.: A61K 7/13

(54) **Hair dye fixatives, hair dyes and hair dyeing methods**

(30) Priority: 30.03.2000 JP 2000095023
(71) Applicant: Shiseido Co., Ltd., Tokyo 104-8010 (JP)
(72) Inventor: Kawasoe, Tomoyuki, c/o Shiseido Research Center, Yokohama-shi, Kanagawa 224-8558 (JP); Ochiai, Masatoshi, c/o Shiseido Research Center, Yokohama-shi, Kanagawa 224-8558 (JP)
(74) Representative: Henkel, Feiler, Hänzel

(57) **Abstract**

The present invention provides a hair dye fixative comprising a complex nucleus capable of forming a complex with an acid dye, a hair dye using said hair dye fixative and a hair dyeing method having an extremely low skin irritating effect, high hair dyeing performance without giving any damage on a hair. By addition of xyloglucan, silicone and an acid in said hair dye fixative and said hair dye, high stability and handling performance, smooth touch of hair after applied and much improvement of dyeing ability and color-sustaining ability are obtained.

## Description

### RELATED APPLICATIONS

This application claims the priority of Japanese Patent Application No. 2000-95023 filed on March 30, 2000, which is incorporated herein by reference.

### FIELD OF THE INVENTION

The present invention relates to a hair dye fixative, a hair dye and a hair dyeing method, particularly to an improvement in the fixation mechanism of an acid dye in a hair.

### BACKGROUND OF THE INVENTION

Examples of a conventional commercial product for changing the color of a hair are an oxidation hair dye employing an oxidation dye (permanent hair dye), an acid hair dye employing an acid dye such as a hair manicure or a color rinse (semi-permanent hair dye) and a temporary hair dye employing a pigment. Among these products, an oxidation hair dye and an acid hair dye are employed to respond to the need of changing the hair color for a prolonged period. Since an oxidation hair dye exhibits a color tone as a result of the polymerization of a low molecular dye precursor within a hair, it has a potent hair dyeing ability and an advantageous property experienced as a sustained color for a prolonged period. However, it requires a patch test for any irritating effect on a head skin, and there is an interval of about 48 hours before dyeing and which leads to a difficulty in performing a quick and simple dyeing, and it also gives a damage on a hair such as an intra-hair S-S bond cleavage.

On the contrary, an acid hair dye can readily be used without any patch test since it accomplishes a hair dyeing by binding an positive-charged amino acid residue within a hair to the acid dye via an ion bond, and it is advantageous also in terms of a color variation and no damage on a hair.

A pH of an acid hair dye is usually needed to be in the range of 1.5 to 4.5 when the hair dye is used. Not to bring an hair dye of such a high acidity in contact with an object except for hair by dropping when using the hair dye, a thickner is generally contained in an acid hair dye. For example thickners such as xanthan gum (Japanese Patent Publication Hei 2-32253), carboxyvinylpolymer and cellulose derivatives such as hydroxyethyl cellulose are generally used for an acid hair dye.

Nevertheless, an acid hair dye undergoes an ion bond cleavage within a hair upon shampooing to allow the dye to be released from the hair, resulting in a difficulty in keeping a finished hair color for a prolonged period, with the period of a sustained color being as problematically short as 1 to 2 weeks.

On the other hand thickners which can be used in a composition of high acidity such as an acid hair dye are considerably limited in number. Moreover it cannot be said that thickners which can be used in high acidity condition are always satisfactorily used for an hair dye.

### SUMMARY OF THE INVENTION

An objective of the present invention, in view of the problems associated with the prior art described above, is to provide a hair dye fixative, a hair dye and a hair dyeing method having an extremely low skin irritating effect, having a high hair dyeing performance without giving any damage on a hair and having a high usability performance, a high stability performance and smooth touch of a hair after used.

Thus, a hair dye fixative according to the present invention comprises a complex nucleus capable of forming a complex with an acid dye.

In this fixative, the complex nucleus is preferably a multivalent metal ion.

Also in this fixative, the complex nucleus is preferably aluminum ion.

Also in this fixative, a combination amount of a complex nucleus is preferably 0.1 to 20 % by weight in terms of the corresponding metal salt.

Also in this fixative, an acid is preferably comprised. An acid comprised is preferably an organic acid, more preferably α -hydroxyl acid and further preferably glycolic acid. A combination amount of an acid is preferably 0.01 to 15 % by weight.

Also in this fixative, silicone is preferably comprised.

Also in this fixative, one or more compounds selected from ethanol, isopropanol, n-propanol, n-butanol and isobutanol is preferably comprised.

A one-component hair dye according to the present invention comprises:
an acid dye;
a complex nucleus capable of forming a complex with said acid dye; and,
an organic solvent having quantitative and qualitative characteristics which do not allow said acid dye to form a dye complex with said complex nucleus.

Also in this hair dye, the complex nucleus is preferably a multivalent metal ion.

Also in this hair dye, the complex nucleus is preferably aluminum ion.

Also this hair dye, a combination amount of a complex nucleus is preferably 0.1 to 20 % by weight in terms of the corresponding metal salt.

Also in this hair dye, xyloglucan is preferably comprised. A combination amount of xyloglucan is preferably 0.1 to 30 % by weight.

Also in this hair dye, silicone is preferably comprised.

Also in this hair dye, an acid is preferably comprised. An acid comprised is preferably an organic acid, more preferably α-hydroxy acid and further preferably glycolic acid. A combination amount of an acid is preferably 0.01 to 15 % by weight.

Also in this hair dye, an aromatic alcohol is preferably comprised.

Also in this hair dye, one or more compounds selected from ethanol, isopropanol, n-propanol, n-butanol and isobutanol is preferably comprised.

A mixing type hair dye wherein a mixture of two compositions is prepared before use and the mixture is applied to hair according to the present invention consists of:
a first composition comprising an acid dye; and,
a second composition comprising a complex nucleus capable of forming a complex with said acid dye.

Also in this hair dye, said complex nucleus is preferably a multivalent metal ion.

Also in this hair dye, said complex nucleus is preferably aluminum ion.

Also in this hair dye, a combination amount of a complex nucleus in a second composition is preferably 0.1 to 20 % by weight in terms of the corresponding metal salt.

Also in this hair dye, xyloglucan is preferably comprised in a first composition. A combination amount of xyloglucan is preferably 0.1 to 30 % by weight.

Also in this hair dye, silicone is preferably comprised in a first and / or second composition.

Also in this hair dye, an acid is preferably comprised in a second composition. An acid comprised is preferably an organic acid, more preferably α -hydroxyl acid and further preferably glycolic acid. A combination amount of an acid is preferably 0.01 to 15 % by weight.

Also in this hair dye, an aromatic alcohol is preferably comprised in a first composition.

Also in this hair dye, benzyl alcohol is preferably comprised in a second composition.

Also in this fixative, one or more compounds selected from ethanol, isopropanol, n-propanol, n-butanol and isobutanol is preferably comprised in a first and / or second composition.

A hair dyeing method according to the present invention is characterized by treating a hair with a hair dye fixative comprising a complex nucleus capable of forming a complex with said acid dye, and then dyeing a hair with an acid dye.

A hair dyeing method according to the present invention is characterized by dyeing a hair with an acid dye and simultaneously treating the hair with a hair dye fixative comprising a complex nucleus capable of forming a complex with said acid dye.

A hair dyeing method according to the present invention is characterized by dyeing a hair with an acid dye, and then treating a hair with a hair dye fixative comprising a complex nucleus capable of forming a complex with said acid dye.

### BRIEF DESCRIPTION OF THE DRAWINGS

Figure 1 shows the color-sustaining ability according to the present invention (value L).
Figure 2 shows the color-sustaining ability according to the present invention (value ΔE).
Figure 3 shows the color-sustaining ability according to the present invention (values a and b).

### DETAILED DISCRIPTION OF THE INVENTION

Preferred embodiments of the present invention are described below.

A complex nucleus employed in the present invention may for example be a trivalent metal salt such as aluminum chloride, a divalent metal salt such as magnesium chloride, calcium chloride and manganese chloride, a monovalent metal salt such as sodium chloride, potassium chloride, lead chloride and zinc chloride, with a di- or trivalent salt being preferred. A metal salt employed may be any metal salt having a complex-forming ability, and sulfates and acetate in addition to the chlorides described above can also give successful results. A metal salt having a complex-forming ability may be employed alone or in combination with each other, and incorporated in an amount of 0.01 to 20.0 % by weight, preferably 0.1 to 10.0 % by weight into the entire composition. An amount less than 0.01 % by weight leads to an extremely low amount used for forming a metal complex within a hair, resulting in a difficulty in obtaining a sufficient hair-dyeing effect and a color tone-sustaining effect. An amount exceeding 20 % by weight is excessive, resulting in a release of the dye from the hair which may lead to a decoloration or a dirty deposition.

An acid dye employed in the present invention may for example be, but not limited to, Amaranth (FD & C Red No.2), Erythrosine (FD & C Red No.3), New Coccin, Rose Bengal, Acid Red, Tartrazine (FD & C Yellow No.5), Sunset Yellow FCF (FD & C Yellow No.6), Fast Green FCF (FD & C Green No.3), Brilliant Blue FCF (FD & C Blue No.1), Indigo Carmine (FD & C Blue No.2), Lithol Rubine B (D & C Red No.6), Fast Acid Magenta (D & C Red No.33), Eosine YS (D & C Red No.22), Eosine YSK, Phloxine BK, Rose Bengal K, Orange II (D & C Orange No.4), Erythrosine Yellowish NA (D & C Orange No.11), Uranine K, Quinoline Yellow WS (D & C Yellow No.10), Alizarine Cyanine Green (FD & C Green No.5), Pyranine Conc (D & C Green No.8), Light Green SF Yellowish, Patent Blue NA, Alphazurine FG (D & C Blue No.4), Dibromofluorescein (D & C Orange No.5), Violamine R (EXT. D & C Red No.3), Ponceau 3R, Ponceau R, Ponceau SX (FD & C Red No.4), Fast Red S, Polar Yellow 5G, Polar Yellow 5G, Naphthol Yellow S (EXT.D & C Yellow No.7), Metanil Yellow (EXT.D & C Yellow No.1), Fast Light Yellow 3G (EXT.D & C Yellow No.3), Naphthol Green B (EXT.D & C Green No.1), Guinea Green B, Alizurol Purple (EXT. D & C Violet No.2), Naphthol Blue Black (D & C Black No.1), an oily dye such as Rhodamine B Stearate (D & C Red No.37), Tetrachlorotetrabromofluorescein (D & C Red No.27), SudanIII (D & C Red No.17), Dibromofluorescein (D & C Orange No.5), Diiodofluorescein (D & C Orange No.10), Fluorescein (D & C Yellow No.7), Quinoline Yellow SS (D & C Yellow No.11), Quinizarine Green SS (D & C Green No.6), Alizurine Purple SS (D & C Violet No.2), Scarlet Red N.F., Oil Red XO, Orange SS (EXT. D & C Orange No.4), Yellow AB, Yellow OB, Sudan Blue B, and other acid dye may also be employed. Among these acid dyes, Tartrazine (FD & C Yellow No.5), Pyranine Conc (D & C Green No.8), Amaranth (FD & C Red No.2), New Coccin, Fast Acid Magenta (D & C Red No.33), Fast Green FCF (FD & C Green No.3), Brilliant Blue FCF (FD & C Blue No.1), Alphazurine FG (D & C Blue No.4), Naphthol Yellow S (EXT.D & C Yellow No.7), Acid Red, Lithol Rubine B (D & C Red No.6), Orange II (D & C Orange No.4), Naphthol Blue Black (D & C Black No.1), Alizarine Cyanine Green (FD & C Green No.5), Alizurol Purple (EXT. D & C Violet No.2) is preferred and Naphthol Blue Black (D & C Black No.1), Alizurol Purple (EXT. D & C Violet No.2), Orange II (D & C Orange No.4), Naphthol Yellow S (EXT. D & C Yellow No.7), Acid Red is particularly preferred.

Any of these acid dyes may be employed alone or in combination with each other, and incorporated in an amount of 0.0001 to 2.0 % by weight, preferably 0.001 to 2.0 % by weight into the entire composition. An amount not more than 0.0001 % by weight leads to an extremely low pigment level within a hair, resulting in an insufficient hair-dyeing effect. An amount exceeding 2.0 % by weight is excessive and may lead to a decoloration and a dirty deposition.

A carrier component for transporting a high molecular weight acid dye into an inner region of a hair may for example be benzyl alcohol.

A solvent for solubilizing benzyl alcohol may for example be ethyl alcohol.

When an acid dye and a complex nucleus are present in an identical composition in the present invention (one-component hair dye), a solvent should be prepared so that the acid dye does not form an insoluble complex with the complex nucleus in this composition. For this purpose, an amount of the acid dye not higher than 2 % by weight in the composition allows a solvent to be prepared appropriately for relevant dye and complex nucleus using an aqueous/alcohol-based solvent.

Xyloglucan is preferably comprised in a hair dye of the present invention. Xyloglucan is not decomposed even in a high acidity condition and is not decomposed by a metal salt. So xyloglucan is extremely stable and an hair dye of the present invention comprising xyloglucan is also stable. So appearances accompanied with a decomposition of thickners such as an increase of pH, a change of viscosity and deterioration of the color and smell does not occur with the passage of the time. Especially in a composition containing a thickner where a metal ion is also contained a stability of the composition is not insufficient even if a thickner generally used for a hair dye is used. But by employing xyloglucan as a thickner excellent stability is obtained.

A composition containing xyloglucan usually shows a thixotropic property. So even when a viscosity of an hair dye of the present invention is established by addition of xyloglucan in a range not to drop from the applied hair, the hair dye has excellent extensibility and applicability. Moreover xyloglucan has little irritation for skin and eye, has no sensitization property, has excellent moisturizing property and can keep soft touch of applied hair.

For such excellent properties of xyloglucan described above, an hair dye of the present invention containing xyloglucan as thickner need not to contain a flow improving agent such as bentonite and bridged polyacrylate like an hair dye containing xanthan gum to improve a flow property and extensibility of the hair dye, and not to drop from hair when using the hair dye.

Though a hair dye containing a flow improving agent has an excellent flow property and excellently prevents the applied hair dye onto hair from dropping, it lacks ease of washing out from hair. But a hair dye of the present invention containing xyloglucan can retain the above-mentioned excellent properties without a flow improving agent.

Xyloglucan has a property that when it is dispersed in a water-rich media its intertwinement between molecular chains is diminished and a viscosity of the solution is relatively low. So an hair dye containing xyloglucan has excellent washing property in that it is easy to wash out from hair after it is applied.

Thus a hair dye of the present invention containing xyloglucan has also excellent safety for the reason that it is easy to wash out from hair after it is applied, and as a result little damage to hair or irritation to skin occurs.

Xyloglucan is now commercially available ( ┌GLYROID ┘ manufactured by DAINIPPON PHARMACEUTICAL CO. LTD.) and widely used as a thickner employed for foods such as sauce or ice cream. When a commercially available xyloglucan is employed for a hair dye of the present invention, a purified grade is preferably used. Xyloglucan can be produced according to the description of the catalogue of ┌GLYROID ┘. Namely xyloglucan is obtained by the process of eliminating aliens from a seed of tamarind, crushing in wet condition, reducing to powder, eliminating inpurities from the resultant, washing and drying, and finally crushing finely.

A combination amount of xyloglucan in a hair dye of the present invention is not limited and a viscosity of the hair dye can be arbitrarily adjusted with a consideration of elements such as extensibility, applicability and dyeing ability onto hair. However a viscosity of a hair dye of the present invention is preferably 5,000 to 200,000 cps when considered these elements. When a viscosity is not more than 5,000 cps the hair dye tends to drop from hair and such a viscosity condition is unpreferable. When a viscosity exceeds 200,000 cps an extension of the hair dye begins to difficult and it tends to lead to deterioration of uniform dyeing property.

Consequently a preferable combination amount of xyloglucan with a hair dye of the present invention is preferably 0.1 to 30 % by weight and more preferably 0.5 to 15 % by weight. In case where a mixing type hair dye wherein a mixture of two compositions is prepared before use and the mixture is applied to hair consisting of an acid hair dye as a first composition comprising xyloglucan and a hair dye fixative as a second composition is used, a combination amount of xyloglucan in said acid hair dye is also preferable to be in the above-mentioned range. In this case, an amount exceeding 30 % by weight per said hair dye may leads to difficulty in mixing said hair dye and said fixative, which is unpreferable.

In the case of two-component hair dye it allowed to combine xyloglucan with the above-mentioned hair dye fixative.

An average molecular weight of xyloglucan is preferably 100,000 to 2,000,000 and more preferably near 650,000.

Other thickners generally employed to an acid hair dye also can be used in the limitation of stability. Such a thickner is preferably an nonionic thickener, such as agar, guar gum, a guar gum derivative such as hydroxypropyl guar gum, a cellulose derivative such as hydroxyethylcellulose and hydroxypropylcellulose.

A thickner such as lauric acid diethanolamide, carboxymethylcellulose, hydroxyethylcellulose, hydroxypropylcellulose, methylcellulose, xanthan gum, carrageenan, alginate salts, pectin, arabic gum, karaya gum, tragacanth gum and agar powder can be comprised in a hair dye of the present invention. But care should be taken not to prevent the effect of the present invention by a combination of these thickners.

It is preferable to contain an acid in a hair dye of the present invention. By the combination of an acid more excellent dyeing ability and color-sustaining ability can be obtained. An organic acid, an inorganic acid and / or their salts can be employed in the hair dye. As an organic acid, for example glycolic acid, lactic acid, tartaric acid, fumaric acid, malic acid, succinic acid, citric acid, levulinic acid, butylic acid, valeric acid, oxalic acid, maleic acid, mandelic acid, pyrrolidone-5-carboxylic acid, glutamic acid, acetic acid, formic acid may be contained in the hair dye. As an inorganic acid, for example phosphoric acid, sulfuric acid, hydrochloric acid, nitric acid may be contained in the hair dye. As these salts, for example, sodium salts, potassium salts, ammonium salts ; triethanolamine salts, diethanolamine salts, monoethanolamine salts such as aminodihydoxymethyl propanediol, 2-amino-2-methyl-1-propanol, 2-amino-2-methyl-1,3-propanediol may be contained in the hair dye.

In these acids an organic acid is preferably used and α-hydroxy acid is more preferably used. As an α-hydroxy acid glycolic acid, lactic acid, tartaric acid, citric acid, fumaric acid and maric acid is preferably used and glycolic acid is more preferably used.

A combination amount of these acids is preferably 0.01 to 15 % by weight, and more preferably 0.1 to 10 % by weight. An amount not more than 0.01 % by weight leads to insufficient effect on dyeing ability and color-sustaining ability. An amount exceeding 15 % by weight leads to unpreferable effect on stability of the hair dye.

In the case of a hair dye fixative of the present invention it is also preferable to combine an acid in the above-mentioned combination amount.

It is preferable to contain a silicone in a hair dye of the present invention. By the combination of a silicone a touch of hair after using the hair dye becomes more improved and damage of hair is effectively prevented.

As a silicone for example dimethylpolysiloxane, methylphenylpolysiloxane, polyether-modified polysiloxane, amino-modified polysiloxane, fluorine-modified polysiloxane, alcohol-modified polysiloxane may be employed. In detail one or more silicones shown in the following may be employed.
(a) Dimethylpolysiloxane of the following formula (1):

   (CH₃)₃SiO[(CH₃)₂SiO]ₖSi(CH₃)₃ (1)

   (In the formula (1) the symbol k means a number of 3 to 650.)
(b) Methylphenylpolysiloxane of the following formula (2) and (3): 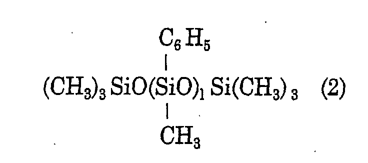 (In the formula (2) the symbol 1 means a number of 1 to 500.)

   (CH₃)₃SiO[(CH₃)₂SiO]ₘ[(C₆H₅)₂SiO]ₙ (3)

   (In the formula (3) the symbol m and n means a number and sum of the number m and n is 1 to 500.)
(c) Polyether-modified polysiloxane of the following formula (4) : 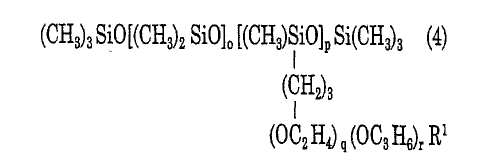 (In the formula (4) the symbol R¹ means a function group selected from hydrogen, alkyl group of C=1 to 12, alkoxy group of C=1 to 6 and hydroxy group. The symbol o means a number of 1 to 100 (preferably 20 to 30) and the symbol p means a number of 1 to 20 (preferably 2 to 10) and the symbol q means a number of 0 to 50 (preferably 20 to 30) and the symbol r means a number of 0 to 50 (preferably 20 to 30))
(d) Amino-modified polysiloxane of the following formula (5) and (6):

   R²(CH₃)₂SiO[(CH₃)₂SiO]ₛ[(OH)(CH₂CH₂CH₂NHCH₂CH₂NH₂)SiO]ₜSi(CH₃)₂R² (5)

   R²(CH₃)₂SiO[(CH₃)₂SiO]ₛ[(CH₃)(CH₂CH₂CH₂NH₂)SiO]ₜSi(CH₃)₂R² (6)

   (In the formula (5) and (6) the symbol R² means a function group selected from hydroxyl group, methyl group and methoxy group. The symbol s means a number of 1 to 20000 and the symbol t means a number of 1 to 50.)
(e) Epoxy-modified polysiloxane of the following formula (7): 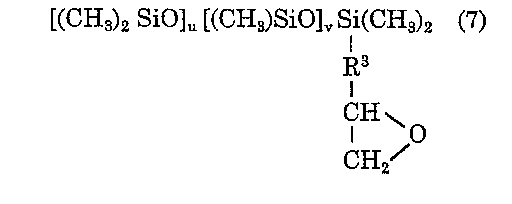 (In the formula (7) the symbol R³ means alkylene group of C=1 to 3. the symbol u means a number of 1 to 500 (preferably 1 to 250) and the symbol v means a number of 1 to 50 (preferably 1 to 30).)
(f) Fluorine-modified polysiloxane of the following formula (8) : 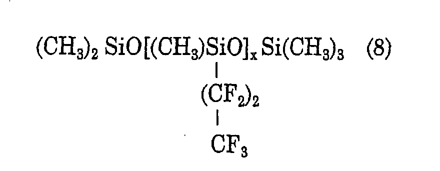 (In the formula (8) the symbol x means a number of 1 to 400 (preferably 1 to 250).)
(g) Alcohol-modified polysiloxane of the following formula (9) and (10):

   OH(CH₂)R⁴[(CH₃)₂SiO]_{y}(CH₃)₂SiR⁴CH₂OH (10)

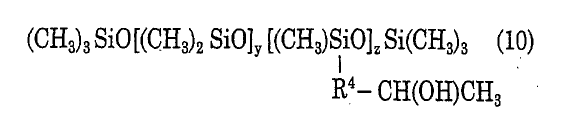

   (In the formula (9) and (10) the symbol R⁴ means alkylene group of C=1 to 4 and R⁴ is allowed to be omitted. The symbol y and z means a number of 1 to 500 (preferably 1 to 200).)
(h) Alkyl-modified polysiloxane of the following formula (11) and (12): 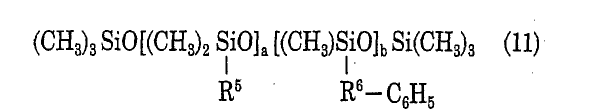 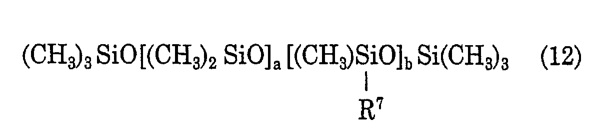 (In the formula (11) and (12) the symbol R⁵ means alkyl group of C=2 to 18. The symbol R⁶ means alkylene group of C=1 to 4 and allowed to be omitted. The symbol R⁷ means alkyl group of C=10 to 16. the symbol a and b means a number of 1 to 500 (preferably 1 to 200).)
(i) A silicone polymer of the following formula (13): 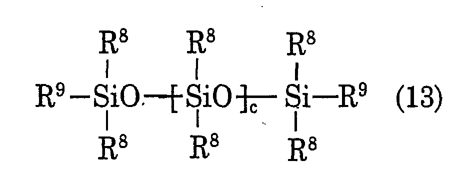 (In the formula (13) the symbol R⁸ means methyl group or partially phenyl group. The symbol R⁹ means methyl group or hydroxy group. The symbol c means a number of 3000 to 20000.)

In the above-mentioned silicones when one or more silicones selected from amino-modified polysiloxane (for example TORAY · SILICONE SM-8702C manufactured by TORAY · SILICONE CO. LTD. ; APS-20EM-735, APS-20DMS and APS-10DMS manufactured by SIN-ETSU CHEMICAL CO. LTD.), silicone polymer (G-40-EM735, G-10-DMS, G-20-DMS manufactured by SIN-ETSU CHEMICAL CO. LTD.), methylphenyl polysiloxane (for example SILICONE KF56 manufactured by SIN-ETSU CHEMICAL CO. LTD.), dimethylpolysiloxane · methyl (polyoxyethylene) siloxane copolymer of average addition mole number 20 to 30 (for example SILICONE SC-9450 manufactured by SIN-ETSU CHEMICAL CO. LTD.) are used, it is not only effective on improvement of hair touch and dyeing ability, but also effective on stability of the hair dye.

As a silicone polymer of the above formula (13), for example, dimethylpolysiloxane rubber manufactured by SIN-ETSU CHEMICAL CO. LTD. (A degree of polymerization (the symbol c in the formula (13)) is 5000 to 8000) may be used.

A combination amount of a silicone in a hair dye of the present invention is preferably 0.01 to 5.0 % by weight. An amount not more than 0.01 % by weight leads to insufficient effect on hair touch. And an amount exceeding 5.0 % by weight occasionally leads to sticky hair touch, which is unpreferable.

In a hair dye of the present invention an organic solvent may be comprised for the purpose of an improvement of usability, solubilization or preventing a formation of an insoluble complex. Such an organic solvent may be, for example, ethanol, isopropanol, n-propanol, n-butanol, isobutanol, ethyleneglycol, propyleneglycol, 1,3-butanediol, dipropyleneglycol, hexyleneglycol, isopreneglycol, glycerine, methylcarbitol, propylcarbitol, butylcarbitol, triethyleneglycol monobutylether, N-methyl-2-pyrrolidone, N-octyl-2-pyrrolidone, N-lauryl-2-pyrrolidone, propylene carbonate, ethylene carbonate. In these organic solvents ethanol, isopropanol, n-propanol, n-butanol is preferable. A preferable combination amount of these organic solvents is 0.5 to 40 % by weight per a hair dye, more preferably 1 to 30 % by weight.

In addition to these organic solvents, an aromatic alcohol such as benzyl alcohol, phenylethyl alcohol, γ -phenylpropyl alcohol, cinnamyl alcohol, anisalcohol, p-methylbennzyl alcohol, α -dimethyl phenylethyl alcohol, α -phenyl ethanol, phenoxy ethanol and benzyloxy ethanol may be employed especially for its properties to carry an acid dye inside the hair. A preferable combination amount of an aromatic alcohol is 0.5 to 20 % by weight per a hair dye, more preferably 1 to 12 % by weight.

In a hair dye of the present invention various agents such as a surfactant, a moisturizing agent, an oil ingredient, a higher alcohol, an emulsifier, a conditioning agent, a fat, a high fatty acid ester, a bactericide, a perfume, a UV-scattering agent, a protein derivative, a plant extract and a medical ingredient can be contained.

As a surfactant, for example, polyoxyethylene surfactants such as polyoxyethylene alkylether, polyoxyethylene fatty acid ester, polyoxyethylenepolyalcohol partially esterified by fatty acid, polyoxyethylene hydrogenated castor oil derivatives ; polyglycerine surfactants such as alkylpolyglycoside like octylpolyglycoside, polyglycerine fatty acid ester, polyglycerine alkyl ether ; nonionic surfactants such as sugar alcohol ethers like maltitol hydroxyalkylether and sorbitol alkylether, fatty acid diethanolamides ; anionic surfactants such as higher fatty acid salts, phosphates, alkyl sulfates, polyoxyethylene alkyl sulfates ; cationic surfactants such as amino acids, alkyl trimethylammonium salts, dialkyl dimethylammonium salts, alkyl dimethyl amine oxides can be contained in a hair dye of the present invention. And amphipathic compound or a surfactant such as stearyl trimethyl ammonium chloride, glycerine monostearate can be contained as a dispersant, or solubilizer in the hair dye.

As a moisturizing agent, for example, glycerine, polyethylene glycol, chondroitin sulfate, hyaluronate, diglycerine, sorbitol, maltitol, pyrrolidone carboxylic acid, lactose, oligosaccharide can be contained in a hair dye of the present invention.

As an oil ingredient, for example, lanolin, squalane, liquid petrolatum, petrolatum, higher fatty acids, triglycerides, ester oil can be contained in a hair dye of the present invention.

As a higher alcohols benzyl alcohol, 2-ethylhexyl alcohol, 2-hexyldecyl alcohol, 2-decyltetradecyl alcohols, isostearyl alcohol, cetostearyl alcohol, lauryl alcohol, oleyl alcohol, myristyl alcohol, palmityl alcohol, stearyl alcohol, cetyl alcohol can be contained in a hair dye of the present invention.

Further, hydrolyzed proteins and their quatery ammonium salts such as hydrolyzed collagen, hydrolyzed keratin, hydrolyzed silk protein, hydrolyzed elastin, hydrolyzed soy peptide ; sequestering agents and antiseptics such as phenacetin, hydroxyethane disulfonic acid and its salts, ethylenediamine tetraacetic acid and its salts, paraben, stannate ; cationic polymer such as poly (dimethylallyl ammonium halide) type cationic polymer, cationic polymer of a condensation product by polyethylene glycol, epichlorohydrin, propylene amine and tallowoyl amine obtained by tallow fatty acid, cationic polymer of a condensation product by polyethylene glycol, epichlorohydrin, propylene amine and cocoyl amine obtained by coconut fatty acid, cationic copolymer of vinylpyrrolidone and dimethylamino methacrylate, cationic cellulose polymer can also be contained in a hair dye of the present invention.

A hair dye of the present invention can be a form of a mixing type hair dye wherein a mixture of two compositions is prepared before use and the mixture is applied to hair consists of a first composition comprising an acid dye and a second composition comprising a complex nucleus capable of forming a complex with said acid dye. A hair dye of this type is especially preferable when a thickner is combined with a hair dye because a decomposition of the thickner by a metal ion as a complex nucleus can be prevented and as a result viscosity stability is improved.

A hair dyeing method according to the present invention is characterized by treating a hair with a hair dye fixative comprising a complex nucleus capable of forming a complex with said acid dye, and then dyeing a hair with an acid dye. A hair dyeing procedure can be conducted simultaneously with treating the hair with a hair dye fixative comprising a complex nucleus capable of forming a complex with said acid dye. And a hair dyeing method in which a hair is dyed with an acid dye, and then a hair is treated with a hair dye fixative comprising a complex nucleus capable of forming a complex with said acid dye, is also probable

An hair dye fixative of the present invention can be formulated into a composition of any product type. For example, the fixative may be in the forms of mist type, spray type, liquid, gel, cream and the like.

An acid hair dye of the present invention can be formulated into a composition of any product type. For example, the acid hair dye may be in the forms of liquid, gel, foam, cream and the like.

### EXAMPLES

The present invention is further described in the following Examples, which are not intended to restrict the invention. An amount is represented as % by weight unless otherwise specified.

### [Evaluation method]

The evaluation method employed in Examples is described below. Thus, an acid hair dye obtained in the form of a gel or a liquid was examined for (1) dyeing ability, (2) touch, (3) secondary deposition and (4) color-sustaining ability.

### (1) Dyeing ability

About 2 g of a Caucasian hair strand was coated evenly with 0.5 g of a sample to effect a dyeing treatment for 15 minutes, followed by shampooing, rinsing and drying, and a resultant dyed strand was subjected to a visual evaluation by 20 experienced panelists based on the comparison with a non-dyed Caucasian hair strand. In this evaluation, a mean score was obtained from the respective scores of the following evaluation items and assigned to one of the 4 grades shown below.
+3: Extremely good
+2: Good
+1: Slightly good
0: Moderate
-1: Slightly poor
-2: Poor
-3: Extremely poor
Assignment of mean score
ⓞ: +2 or higher
○: + 1 or higher and less than +2
Δ: -1 or higher and less than +1
× : Less than -1

### (2) Touch

About 2 g of a Caucasian hair strand was coated evenly with 0.5 g of a sample to effect a dyeing treatment for 15 minutes, followed by shampooing, rinsing and drying, and a resultant dyed strand was subjected to a touch evaluation by 20 experienced panelists. The evaluation was performed as described above for the dyeing ability.

### (3) Secondary deposition

About 2 g of a Caucasian hair strand was coated evenly with 0.5 g of a sample to effect a dyeing treatment for 15 minutes, followed by shampooing and rinsing, and a resultant wet dyed strand was placed on a white towel for 20 minutes and then subjected to an evaluation by 20 experienced panelists for the deposition of the color on the white towel. The evaluation was performed as described above for the dyeing ability.

### (4) Color-sustaining ability

About 2 g of a Caucasian hair strand was coated evenly with 0.5 g of a sample to effect a dyeing treatment for 15 minutes, followed by shampooing, rinsing and drying to obtain a dyed strand. The strand thus obtained was shampooed with a commercially available shampoo each for 1 minutes 7 times repetitively, and then the color tone of the strand was evaluated by 20 experienced panelists. The evaluation was performed as described above for the dyeing ability.

### [One-component hair dye]

The results of the evaluation of one-component hair-dyeing gels are shown in Table 1.

**Table 1**

| | Sample 1 | 2 | 3 | 4 | 5 | 6 | 7 | 8 |
|---|---|---|---|---|---|---|---|---|
| (1) Nonionic thickner | 2.0 | 2.0 | 2.0 | 2.0 | 2.0 | 2.0 | 2.0 | 2.0 |
| (2) Benzyl alcohol | 8.0 | 8.0 | 8.0 | 8.0 | 8.0 | 8.0 | 8.0 | 8.0 |
| (3) Naphthol Blue Black (D & C Black No.1) | 0.2 | 0.2 | 0.2 | 0.2 | 0.2 | 0.2 | 0.2 | 0.2 |
| (4) Naphthol Yellow S (EXT.D & C Yellow No.7) | 0.1 | 0.1 | 0.1 | 0.1 | 0.1 | 0.1 | 0.1 | 0.1 |
| (5) Fast Acid Magenta (D & C Red No.33) | 0.1 | 0.1 | 0.1 | 0.1 | 0.1 | 0.1 | 0.1 | 0.1 |
| (6) Ethanol | 20.0 | 20.0 | 20.0 | 20.0 | 20.0 | 20.0 | 20.0 | 20.0 |
| (7) Aluminum chloride | 0.0 | 0.005 | 0.01 | 0.1 | 1.0 | 10.0 | 20.0 | 25.0 |
| (8) Perfume | 0.1 | 0.1 | 0.1 | 0.1 | 0.1 | 0.1 | 0.1 | 0.1 |
| (9) Ion-exchange water | B.L.(#1). | B.L | B.L. | R.L. | B.L. | B.L. | B.L. | B.L. |
| Dyeing ability | Δ | Δ | ○ | ⓞ | ⓞ | ⓞ | ○ | Δ |
| Touch | ⓞ | ⓞ | ⓞ | ⓞ | ⓞ | ⓞ | ⓞ | ⓞ |
| Secondary deposition | ⓞ | ⓞ | ⓞ | ⓞ | ⓞ | ⓞ | ⓞ | ⓞ |
| Color-sustaining ability | Δ | Δ | ○ | ⓞ | ⓞ | ⓞ | ○ | Δ |
| (#1): The symbol B.L. means balance. | | | | | | | | |

In the experiments, Components (1) and (7) were dissolved completely in Component (9), which was combined with Component (6) with which Components (2) and (8) were mixed uniformly. A resultant solution was combined with a part of Component (9) in which Components (3), (4) and (5) were dissolved to obtain an acid hair-dyeing gel.

As evident from Table 1, aluminum chloride as a complex nucleus began to exhibit its effect at 0.01 % and the effect came to be higher particularly at a concentration of 0.1 to 10 %, but then acted rather adversely at a concentration higher than 20 %. This behavior may be attributable to the complex formation as a result of the evaporation even of a small amount of an alcohol, whereby depositing a complex on the surface of a hair and preventing a migration into the inside of the hair. Accordingly, a preferable amount of a complex nucleus to be incorporated is about 0.01 to 20 %, more preferably 0.1 to 10 %.

A similar experiment was conducted also with a temporary hair dye. The results are shown in Table 2.

**Table 2**

| | Sample 9 | 10 |
|---|---|---|
| (1) Carbon black | 1.0 | 1.0 |
| (2) Acrylic resin alkanol amine solution (50 %) | 6.0 | 6.0 |
| (3) Aluminum chloride | 0.1 | ― |
| (4) Ethanol | Balance | Balance |
| Dyeing ability | Δ | Δ |
| Touch | ⓞ | ⓞ |
| Secondary deposition | × | × |
| Color-sustaining ability | × | × |

As evident from Table 2, absolutely no effect of the addition of aluminum chloride on a temporary hair dye employing a pigment such as a carbon black was observed. This means that the interaction between an acid dye and aluminum chloride indicated in Table 1 shown above is extremely specific.

Accordingly, we then made a formulation from an acid dye (0.4%), aluminum chloride · 6H₂O (0.5 %), ethanol (20 %), benzyl alcohol (8 %) and water (balance) according to the method for producing the one-component hair dye described above. The formulation was placed in a flask and the solvent was evaporated using a rotary evaporator under reduced pressure at 50°C. Although a precipitation was formed while the evaporation of the solvent was proceeded, no complete removal of the solvent was accomplished even after the evaporation for about 1 hour and a non-volatile solvent associated with the precipitate was obtained. This was then combined with various solvents in an attempt to effect a dissolution. As a result, partial dissolution in water and ethanol, slight dissolution in acetone and almost no dissolution in hexane were noted, but a complete dissolution in 0.4 % EDTA · 3Na was accomplished. Accordingly, the precipitate formed upon removal of the solvent was assumed to be a metal complex with the dye.

Based on the findings described above, we considered that when a one-component hair dye according to the present invention is applied to a hair an acid dye can migrate as usual into a cuticle and a part of a cortex where coexisting aluminum ion and the acid dye forms an insoluble complex upon evaporation of the solvent. Thus, an apparent molecular weight becomes larger and it becomes possible to prevent the pigment from being released from the hair.

Subsequently, we prepared red-shaded hair dyes having the compositions in Table 3, which were subjected to a more detailed examination for the color-sustaining ability.

**Table 3**

| | Sample 11 | 12 |
|---|---|---|
| (1) Nonionic thickner | 2.0 | 2.0 |
| (2) Benzyl alcohol | 8.0 | 8.0 |
| (3) Fast Acid Magenta (D & C Red No.33) | 0.2 | 0.2 |
| (4) Ethanol | 20.0 | 20.0 |
| (5) Aluminum chloride | 0.5 | ― |
| (6) Perfume | 0.1 | 0.1 |
| (7) Ion-exchange water | Balance | Balance |

### Evaluation of color-sustaining ability

About 2 g of a Caucasian hair strand was coated evenly with 0.5 g of a sample to effect a dyeing treatment for 15 minutes, followed by shampooing with or without rinsing and then drying to obtain a dyed strand. The strand thus obtained was shampooed with a commercially available shampoo each for 1 minutes, and then the color tone of the strand was analyzed using a colorimeter. This procedure was repeated seven times. Sample 13 was dyed similarly to Sample 11 and then allowed to stand for 15 minutes prior to the shampooing described above.

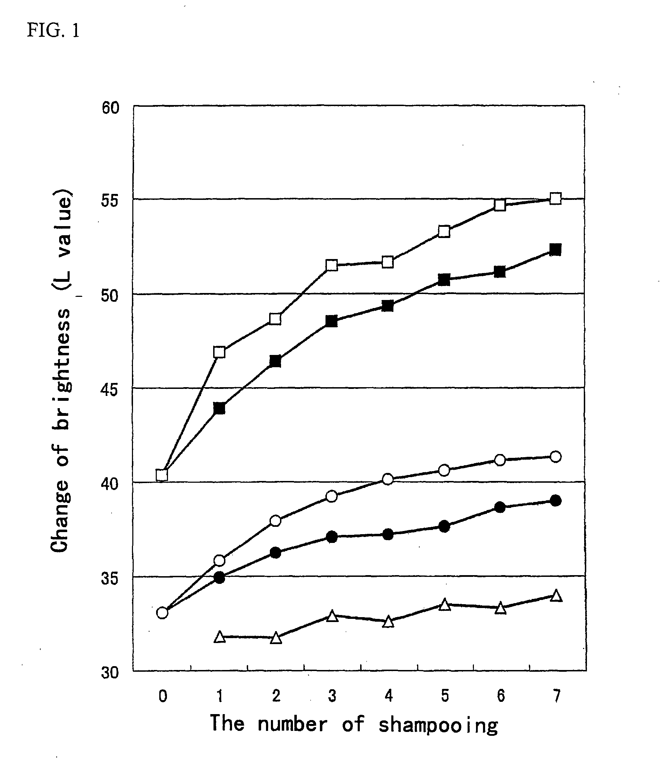

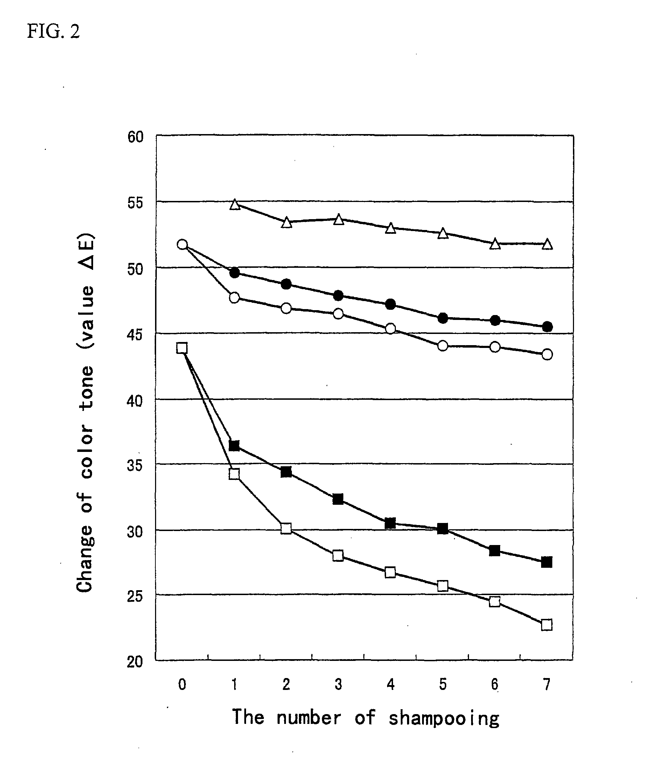

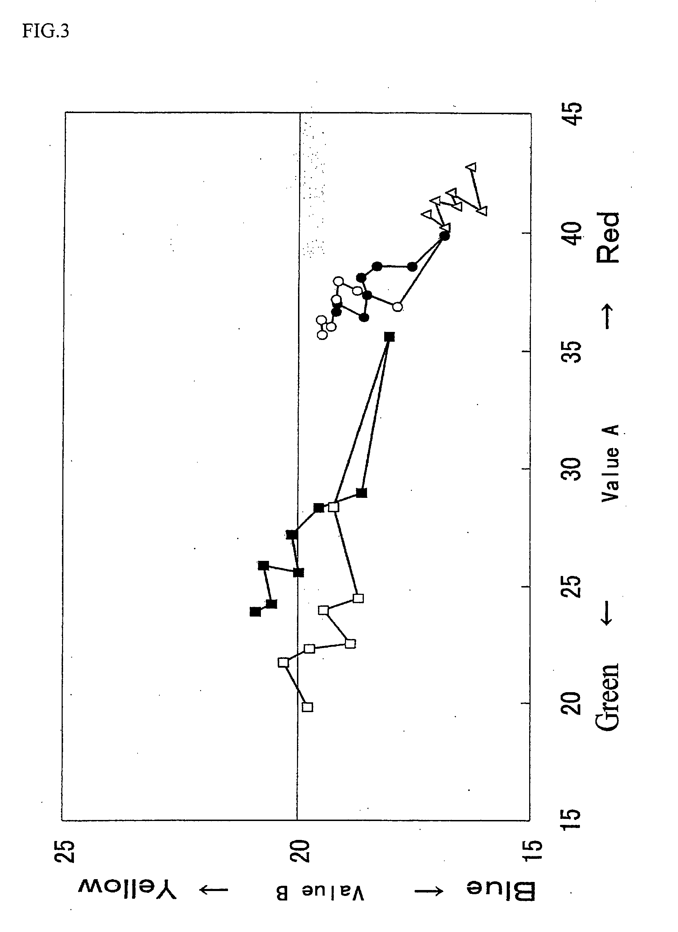

The results are shown in Figures 1 to 3.
■: Sample 12 - Shampooing
□: Sample 12 - Shampooing, rinsing
•: Sample 11 - Shampooing
○: Sample 11 - Shampooing, rinsing
Δ: Sample 13 - Shampooing

As evident from Figures, Sample 11 exhibited not only a higher dyeing ability when compared with Sample 12 but also a markedly excellent ability of sustaining the color tone in spite of repetitive shampooing. Also as evident from the results of Sample 13, a time interval provided after the dyeing treatment served to further enhance the color-sustaining ability.

### [Fixative]

Subsequently, we investigated the fixative effect of an aqueous solution of aluminum chloride when applying after using a hair dye employing an acid dye.

### Hair dye composition (1)

| | |
|---|---|
| (1) Nonionic thickner | 2.0 |
| (2) Benzyl alcohol | 8.0 |
| (3) Naphthol Blue Black (D & C Black No.1) | 0.2 |
| (4) Naphthol Yellow S (EXT. D & C Yellow No.7) | 0.1 |
| (5) Fast Acid Magenta (D & C Red No.33) | 0.1 |
| (6) Ethyl alcohol | 20.0 |
| (7) Perfume | 0.1 |
| (8) Ion-exchange water | Balance |

The hair dye having the composition shown above was used to dye a hair as described above, and then a fixative (mist type) having a composition shown below was sprayed onto a strand. After allowing to stand for 20 minutes followed by drying, each strand was examined for the difference in the color (ΔE before washed) from the strand before dyeing. Subsequently, the strand was shaken together with 100 ml of a 2% aqueous solution of sodium lauryl sulfate in a conical flask at 30°C for 1 hour. After washing with water followed by drying, ΔE between each strand and the respective strand before dyeing (ΔE of a washed strand) and ΔE of the respective washing were determined. The results are shown in Table 4.

**Table 4**

| Fixative composition (mist type) | | | | |
|---|---|---|---|---|
| | Sample 14 | 15 | 16 | 17 |
| Aluminum chloride hexahydrate | 0 | 0.4 | 1.0 | 5.0 |
| Ion-exchanged water | B.L.(#1) | B.L. | B.L. | B.L. |
| ΔE of a washed strand | 37.702 | 42.832 | 43.139 | 44.293 |
| ΔE before washed | 45.562 | 46.680 | 45.716 | 46.836 |
| ΔE loss | 7.860 | 3.848 | 2.577 | 2.543 |
| ΔE of washing | 38.513 | 30.328 | 29.691 | 25.321 |

| | | | | |
|---|---|---|---|---|
| (#1): The symbol B.L. means balance. | | | | |

Based on the results shown in Table 4, aluminum chloride was proven to have an excellent effect even when being used as a fixative separately from a hair dye.

Subsequently, we investigated a preferable combination amount of a complex nucleus by employing a fixative composition (mist type).

| Hair dye composition (2) | |
|---|---|
| (1) Carboxyvinylpolymer | 3.0 |
| (2) Benzyl alcohol | 8.0 |
| (3) Orange II (D & C Orange No.4) | 0.4 |
| (4) New Coccin | 0.1 |
| (5) Dipropylene glycol | 15.0 |
| (6) Sodium hydroxide | 0.03 |
| (7) Ion-exchange water | Balance |

The hair dye having the composition shown above was used to dye a hair as described above, and then a fixative having a composition shown below was sprayed onto a strand. After allowing to stand for 10 minutes followed by drying, each strand was examined for the difference in the color (ΔE before washed) from the strand before dyeing. Subsequently, the strand was shaken together with 100 ml of a 2% aqueous solution of sodium dodecyl sulfate in a conical flask at 30°C for 10 minutes and this procedure was repeated 3 times. After washing with water followed by drying, ΔE between each strand and the respective strand before dyeing (ΔE of a washed strand) were determined. Then the color-sustaining ability of a sample was evaluated based on a difference between said ΔE before washed and ΔE of a washed strand (ΔE loss) The results are shown in Table 5.

### Color-sustaining ability

ⓞ: ΔE loss was less than 4.
○: ΔE loss was 4 or higher and less than 8.
Δ: ΔE loss was 8 or higher and less than 12.
× : ΔE loss was 12 or higher.

**Table 5**

| Fixative composition (mist type) | | | | |
|---|---|---|---|---|
| | Sample 18 | 19 | 20 | 21 |
| Aluminum chloride hexahydrate | 0 | 0.1 | 1.0 | 4.0 |
| Ethanol | 20 | 20 | 20 | 20 |
| Ion-exchange water | B.L.(#1) | B.L | B.L. | B.L. |
| ΔE of a washed strand | 22 | 33 | 35 | 37 |
| ΔE before washed | 40 | 40 | 40 | 40 |
| ΔE loss | 18 | 7 | 5 | 3 |
| Color-sustaining ability | × | ○ | ○ | ⓞ |
| (#1): The symbol B.L. means balance. | | | | |

The mist type fixative above-mentioned was produced by dissolving ethanol in ion-exchange water followed by dissolving aluminum chloride hexahydrate, and filling up the resultant in a mist receptacle.

Based on the results shown in Table 5, the combination effect of aluminum chloride as a complex nucleus began to be obvious at a combination amount of near 0.1 %. Though it is not shown in Table 5, a combination amount higher than 20 % occasionally acts rather adversely. This behavior may be attributable to the complex formation as a result of the evaporation even of a small amount of an alcohol, whereby depositing a complex on the surface of a hair and preventing a migration into the inside of the hair. Accordingly, a preferable amount of a complex nucleus to be incorporated is about 0.1 to 20 %.

Subsequently, we also investigated the cases where the treatment by a fixative is conducted before a hair dye is applied onto hair (treatment before hair dyeing), and the cases where the treatment by a fixative is conducted simultaneously with applying a hair dye onto hair (simultaneous treatment with hair dyeing). A hair dye of said hair dye composition (2) and a hair dye fixative of the following composition in Table 6 (mist type) was used and a hair dyeing ability and a color-sustaining ability were evaluated. A hair dyeing treatment was done as follows.

### Treatment before hair dyeing :

A suitable amount of the hair dye fixative of Sample 23 in

Table 6 was sprayed onto a strand and left for 10 minutes at 30°C. Then a suitable amount of the hair dye was applied and left for 10 minutes at 30°C followed by washing and drying.

### Simultaneous treatment with hair dyeing :

A suitable amount of the hair dye was applied onto a strand followed by spraying a suitable amount of the hair dye fixative of Sample 24 in Table 6. Then the strand was left for 10 minutes at 30°C followed by washing and drying.

For the each case said ΔE before washed and ΔE of a washed strand were determined. Then a dyeing ability and a color sustaining ability were evaluated based on the following evaluation standard.

### Dyeing ability

ⓞ: The difference in said ΔE before washed from that of the case where a treatment by the hair dye fixative was not conducted was 4 or higher.
○: The difference in said ΔE before washed from that of the case where a treatment by the hair dye fixative was not conducted was 1 or higher and less than 4.
× : The difference in said ΔE before washed from that of the case where a treatment by the hair dye fixative was not conducted was less than 1.

### Color-sustaining ability

ⓞ: ΔE loss was less than 4.
○: ΔE loss was 4 or higher and less than 8.
Δ: ΔE loss was 8 or higher and less than 12.
×: ΔE loss was 12 or higher.

The results are shown in Table 6.

**Table 6**

| Fixative composition (mist type) | | | |
|---|---|---|---|
| | Sample 22 | 23 | 24 |
| | | Treatment before hair dyeing | Simultaneous treatment |
| Aluminum chloride hexahydrate | ― | 2.0 | 2.0 |
| Ethanol | ― | 10.0 | 10.0 |
| Benzyl alcohol | ― | 10.0 | 10.0 |
| 1,3-Butylene glycol | ― | 5.0 | 5.0 |
| Dipropylene glycol | ― | 5.0 | 5.0 |
| Glycollic acid | ― | 1.0 | 1.0 |
| Sodium lactate | ― | 0.5 | 0.5 |
| Ion-exchange water | ― | balance | balance |
| ΔE of a washed strand | 28 | 43 | 43 |
| ΔE before washed | 40 | 46 | 46 |
| ΔE loss | 12 | 3 | 3 |
| Dyeing ability | ― | ⓞ | ⓞ |
| Color-sustaining ability | × | ⓞ | ⓞ |

From Table 6 it is obvious that both the case of the treatment by a hair dye fixative before dyeing and the simultaneous treatment by a hair dye fixative with hair dyeing are also shows sufficient effect on dyeing ability and color-sustaining ability.

### [Effect of a combination of xyloglucan on usability and stability]

Subsequently we investigated the effect of a combination of xyloglcan on stability and usability of a hair dye in which xyloglucan is employed as a thickner. The stability and usability was evaluated based on the following standard. The dyeing ability evaluation of (1) and the color-sustaining ability evaluation of (4) which is mentioned above was also conducted.

### Stability

An each sample hair dye shown in Table 7 was left for a week at 70°C. Then pH and viscosity of the sample was measured and a stability was evaluated based on the following standard.
○ : No change of pH and viscosity was recognized after leaving a sample for a week at 70°C relative to those before leaving the sample.
Δ : A change of pH and viscosity was recognized after leaving a sample for a week at 70°C relative to those before leaving the sample. But a degree of the change was in a permissible range for using.
× : A change of pH and viscosity was recognized after leaving a sample for a week at 70°C relative to those before leaving the sample. A degree of the change exceeded a permissible range for using.

### Usability

An each sample hair dye shown in Table 7 was used by 20 panelists and extensibility, applicability and uniform-dyeing ability when an each sample was applied on hair were evaluated based on the following standard.
○ : When a sample was applied onto hair no dropping from the applied hair was occurred and no mass was existed in a sample. It was easy to extend the sample onto hair.
Δ : When a sample was applied onto hair a slight dropping from the applied hair was occurred and a little number of masses were existed in a sample. It was difficult to extend the sample onto hair.
× : When a sample was applied onto hair a considerable dropping from the applied hair was occurred and a great number of masses were existed in a sample. It was extremely difficult to extend the sample onto hair.

The results are shown in Table 7.

**Table 7**

| | Sample 25 | 26 | 27 | 28 | 29 | 30 | 31 | 32 |
|---|---|---|---|---|---|---|---|---|
| (1) Xyloglucan | 0 | 0.1 | 0.5 | 2 | 5 | 15 | 30 | 40 |
| (2) Hydroxyethyl cellulose | 2 | 1.8 | 1 | 0 | 0 | 0 | 0 | 0 |
| (3) Benzyl alcohol | 8 | 8 | 8 | 8 | 8 | 8 | 8 | 8 |
| (4) Naphthol Blue Black (D & C Black No.1) | 0.1 | 0.1 | 0.1 | 0.1 | 0.1 | 0.1 | 0.1 | 0.1 |
| (5) Orange II (D & C Orange No.4) | 0.1 | 0.1 | 0.1 | 0.1 | 0.1 | 0.1 | 0.1 | 0.1 |
| (6) Alizurol Purple (EXT. D & C Violet No.2) | 0.1 | 0.1 | 0.1 | 0.1 | 0.1 | 0.1 | 0.1 | 0.1 |
| (7) Violamine R (EXT. D & C Red No.3) | 0.1 | 0.1 | 0.1 | 0.1 | 0.1 | 0.1 | 0.1 | 0.1 |
| (8) 1,3-butyleneglycol | 15 | 15 | 15 | 15 | 15 | 15 | 15 | 15 |
| (9) Aluminum chloride ·hexahydrate | 0.5 | 0.5 | 0.5 | 0.5 | 0.5 | 0.5 | 0.5 | 0.5 |
| (10) Ion-exchange water | B.L.(#1) | B.L. | B.L. | B.L. | B.L. | B.L. | B.L. | B.L. |
| Stability | × | Δ | ○ | ○ | ○ | ○ | ○ | ○ |
| Usability | ○ | ○ | ○ | ○ | ○ | ○ | Δ | × |
| Dyeing ability | ⓞ | ⓞ | ⓞ | ⓞ | ⓞ | ⓞ | ⓞ | ⓞ |
| Color-sustaining ability | ⓞ | ⓞ | ⓞ | ⓞ | ⓞ | ⓞ | ⓞ | ⓞ |
| (#1): The symbol B.L. means balance. | | | | | | | | |

In the experiments, Components (1) and (2) were dissolved completely in a part of Component (10) at 70°C, which was combined with Component (3), Component (8) and Component (9) dissolved in a part of Component (10) A resultant solution was combined with dyes dissolved in a part of Component (10) to obtain each sample hair dye. In the case of a sample25, 26 and 27 an amount of hydroxyethyl cellulose was adjusted to obtain almost equal viscosity for these samples.

As evident from Table 7, It is effective on usability and stability when a combination amount of xyloglucan was in the range of 0.1 to 30 % by weight. In the case where only hydroxyethyl cellulose was combined as a thickner a viscosity of the sample changed by time and lack a stability for viscosity. It was especially effective on usability and stability when a combination amount of xyloglucan was in the range of 0.5 to 15 % by weight. Accordingly, a preferable amount of xyloglycan to be incorporated is about 0.1 to 30 %, more preferably 0.5 to 15 %.

### [Effect of a combination of acid]

Subsequently we investigated the effect of a combination of an acid on dyeing ability and color-sustaining ability by preparing an one-component hair dye in which an acid was added.

| Hair dye composition (3) | | |
|---|---|---|
| (1) | Hydroxyethyl cellulose | 0.5 |
| (2) | Agar | 2.0 |
| (3) | Benzyl alcohol | 8.0 |
| (4) | Orange II (D & C Orange No.4) | 0.44 |
| (5) | New Coccin | 0.15 |
| (6) | Methyl paraben | 20.0 |
| (7) | Aluminum chloride hexahydrate | 0.5 |
| (8) | 95% Brucine-modified alcohol | 20.0 |
| (9) | an acid (A sort and a combination amount are shown in Table 8.) | |
| (10) | 50 % Sodium lactate (A combination amount are shown in Table 8.) | |
| (11) | Ion-exchange water | Balance |

The dyeing treatment onto a Caucasian hair strand for 10 minutes at 30°C was conducted by employing a hair dye of the composition shown above. After shampooing and drying, each strand was examined for the difference in the color (ΔE before washed) from the strand before dyeing. Subsequently, the strand was shaken together with 100 ml of a 2% aqueous solution of sodium dodecyl sulfate in a conical flask at 30°C for 10 minutes and this procedure was repeated 3 times. After washing with water followed by drying, ΔE between each strand and the respective strand before dyeing (ΔE of a washed strand) were determined.

From each ΔE dyeing ability and color-sustaining ability were evaluated based on the following standard. The results are shown in Table 8.

### Dyeing ability

ⓞ: The difference between ΔE before washing of the case where a treatment by a sample was conducted and that of the case where a treatment by the sample in which an acid was not combined (the sample 33 in Table 8) was conducted was 4 or higher.
○: The difference between ΔE before washing of the case where a treatment by a sample was conducted and that of the case where a treatment by the sample in which an acid was not combined (the sample 33 in Table 8) was conducted was 0 or higher and less than 4.
Δ: The difference between ΔE before washing of the case where a treatment by a sample was conducted and that of the case where a treatment by the sample in which an acid was not combined (the sample 33 in Table 8) was conducted was less than 0.

### Color-sustaining ability

ⓞ: The difference between said ΔE loss of the case where a treatment by the sample in which an acid was not combined (the sample 33 in Table 8) was conducted and that of the case where a treatment by a sample was conducted was 10 or higher.
○: The difference between said ΔE loss of the case where a treatment by the sample in which an acid was not combined (the sample 33 in Table 8) was conducted and that of the case where a treatment by a sample was conducted was 0 or higher and less than 10.
Δ: The difference between said ΔE loss of the case where a treatment by the sample in which an acid was not combined (the sample 33 in Table 8) was conducted and that of the case where a treatment by a sample was conducted was less than 0.

**Table 8**

| | Sample 33 | 34 | 35 | 36 | 37 | 38 | 39 | 40 | 41 |
|---|---|---|---|---|---|---|---|---|---|
| (An acid employed) | | | | | | | | | |
| Citric acid | ― | 0.02 | ― | ― | ― | ― | ― | ― | ― |
| Glycolic acid | ― | ― | 0.1 | 0.2 | 0.4 | 1.0 | 1.6 | 2.4 | 5.0 |

| (pH adjusting agent) | | | | | | | | | |
|---|---|---|---|---|---|---|---|---|---|
| 50 % Sodium lactate | ― | ― | ― | 0.2 | 0.3 | 0.4 | 0.9 | 1.3 | 2.4 |
| pH | 2.8 | 2.3 | 2.3 | 2.3 | 2.3 | 2.3 | 2.3 | 2.3 | 2.3 |
| ΔE before washing | 40 | 42 | 46 | 46 | 49 | 51 | 51 | 51 | 51 |
| ΔE of a washed strand | 28 | 34 | 40 | 42 | 42 | 44 | 45 | 45 | 45 |
| Dyeing ability | ― | ○ | ⓞ | ⓞ | ⓞ | ⓞ | ⓞ | ⓞ | ⓞ |
| Color-sustaining ability | ― | ○ | ⓞ | ⓞ | ⓞ | ⓞ | ⓞ | ⓞ | ⓞ |

In the experiments, Components all (3) to (10) were dissolved completely in a part of Component (11) and the resultant was mixed with Component (1) and Component (2) dissolved in a part of Component (11) to obtain each sample hair dye.

As evident from Table 8, an addition of an acid in a hair dye of the present invention comprising an acid dye and a complex nucleus which is capable of forming a complex with the acid dye was effective on a dyeing ability and a color-sustaining ability. Although the results are not shown in Table 8, an amount less than 0.01 % by weight leads to insufficient effect on a dyeing ability and a color-sustaining ability, and an amount exceeding 15 % by weight leads to give unpreferable effect on a stability of the hair dye. A combination amount of 0.1 to 10 % by weight was especially effective on a dyeing ability and a color-sustaining ability. Accordingly, a preferable amount of an acid to be incorporated is about 0.01 to 15 %, more preferably 0.1 to 10 %.

Subsequently we prepared an one-component hair dye of the following composition containing an acid and investigated a relation between a sort of an acid incorporated and a dyeing ability and a color-sustaining ability of the sample hair dye based on said ΔE before washing, said ΔE of a washed strand and a difference between these two parameters (ΔE loss). The results are shown in Table 9.

| Hair dye composition (4) | | |
|---|---|---|
| (1) | Hydroxyethyl cellulose | 0.5 |
| (2) | Agar | 2.0 |
| (3) | Benzyl alcohol | 8.0 |
| (4) | Naphthol blue black (D & C Black No.1) | 0.2 |
| (5) | Alizurol Purple (EXT. D & C Violet No.2) | 0.1 |
| (6) | Orange II (D & C Orange No.4) | 0.4 |
| (7) | Methyl paraben | 0.1 |
| (8) | Aluminum chloride hexahydrate | 0.5 |
| (9) | 95% Brucine modified alcohol | 20.0 |
| (10) | an acid (A sort and a combination amount are shown in Table 9.) | |
| (11) | 50 % Sodium lactate (A combination amount are shown in Table 9.) | |
| (12) | Ion-exchange water | Balance |

**Table 9**

| | Sample 42 | 43 | 44 | 45 | 46 |
|---|---|---|---|---|---|
| (An acid employed) | | | | | |
| Citric acid | ― | 1.6 | ― | ― | ― |
| Tartaric acid | ― | ― | 1.6 | ― | ― |
| Lactic acid | ― | ― | ― | 1.6 | ― |
| Glycolic acid | ― | ― | ― | ― | 1.6 |

| (pH adjusting agent) | | | | | |
|---|---|---|---|---|---|
| 50 % Sodium lactate | ― | 1.1 | 1.1 | 0.9 | 0.9 |
| pH | 2.8 | 2.3 | 2.3 | 2.3 | 2.3 |
| ΔE before washing | 40 | 44 | 48 | 51 | 55 |
| ΔE of a washed strand | 28 | 36 | 40 | 44 | 51 |
| ΔE loss | 12 | 8 | 8 | 7 | 4 |

As evident from Table 9, when α -hydroxy acid such as tartaric acid, lactic acid and glycollic acid was employed the effect on a dyeing ability and a color-sustaining ability by incorporating acid is excellent, and when glycollic acid was employed the effect was especially excellent.

### [Examples]

Preferred formulations according to the present inventions are described below.

| Formulation 1 : Color rinse (one-component type) | |
|---|---|
| Xyloglucan | 2.0 |
| Naphthol Blue Black (D & C Black No.1) | 0.01 |
| Orange II (D & C Orange No.4) | 0.01 |
| Alizurol Purple (EXT. D & C Violet No.2) | 0.01 |
| Fast Acid Magenta (D & C Red No.33) | 0.01 |
| Benzyl alcohol | 8.0 |
| Aluminum Chloride hexahydrate | 0.1 |
| 1,3-Butylene glycol | 15 |
| Cetanol | 1.0 |
| α-Olefinsulfonic acid | 0.5 |
| Polyoxyethylene hydrogenated castor oil | 0.5 |
| Amino-modified silicone | 0.5 |
| (This is an amino-modified siloxane of the above-mentioned general formula (6). In the formula R² is methyl group and s=10000, t=10.) | |
| Lactic acid | 0.1 |
| Sodium lactate | 0.1 |
| Ion-exchange water | Balance |

### <Production method>

Cetanol is added to 1,3-butylene glycol and dissolved with heating at 70°C and then combined with α-olefinsulfonic acid and polyoxyethylene hydrogenated castor oil (organic phase). Separately, aluminum chloride, lactic acid and sodium lactate are dissolved in an ion-exchange water followed by dissolution of xylogulucan at 70°C (aqueous phase). To the organic phase, the aqueous phase is added followed by benzyl alcohol and finally a dye dissolved in an ion-exchange water, and mixed.

The color rinse thus obtained exhibited excellent dyeing ability, color-sustaining ability, handling performance and stability. It also exhibited uniform dyeing, no dropping from hair, ease of washing out after applying onto hair, touch and had a characteristic of soft touch.

| Formulation 2 : Hair dye (one-component type) | |
|---|---|
| Xyloglucan | 7.0 |
| Xanthangum | 0.5 |
| Naphthol Blue Black (D & C Black No.1) | 0.2 |
| Alizurol Purple (EXT. D & C Violet No.2) | 0.3 |
| Sunset Yellow FCF (FD & C Yellow No.6) | 0.1 |
| Benzyl alcohol | 5.0 |
| Aluminum Chloride hexahydrate | 0.4 |
| Dimethylpolysiloxane | 0.5 |
| (This is a silicone polymer of the above-mentioned general formula (13). In the formula R⁸ and R⁹ are methyl group and c=10000.) | |
| Tetrahydrofurfuryl alcohol | 12.0 |
| Glycerine | 0.5 |
| Polyoxyethylene (100) hydrogenated castor oil | 1.0 |
| Hydrolyzed silk protein | 0.1 |
| Lactic acid | 2.0 |
| Perfume | Suitable amount |
| Ion-exchange water | Balance |

### <Production method>

The production method was in accordance with a standard method.

The hair dye thus obtained exhibited excellent dyeing ability, color-sustaining ability, handling performance and stability. It also exhibited uniform dyeing, no dropping from hair, ease of washing out after applying onto hair, touch and had a characteristic of soft touch.

| Formulation 3 : Hair dye (one-component) | |
|---|---|
| Xyloglucan | 4.0 |
| Naphthol Blue Black (D & C Black No.1) | 0.2 |
| Alizurol Purple (EXT. D & C Violet No.2) | 0.3 |
| Sunset Yellow FCF (FD & C Yellow No.6) | 0.1 |
| Benzyl alcohol | 5.0 |
| Magnesium chloride dihydrate | 0.4 |
| Dimethylpolysiloxane | 0.5 |
| (This is a dimethylpolysiloxane of the above-mentioned general formula (1). In the formula k=5 to 20.) | |
| Tetrahydrofurfuryl alcohol | 12.0 |
| Glycerine | 0.5 |
| Polyoxyethylene (100) hydrogenated castor oil | 1.0 |
| Hydrolyzed elastin | 0.2 |
| Lactic acid | 2.0 |
| Perfume | Suitable amount |
| Ion-exchange water | Balance |

### <Production method>

The production method was in accordance with a standard method.

The hair dye thus obtained exhibited excellent dyeing ability, color-sustaining ability, handling performance and stability. It also exhibited uniform dyeing, no dropping from hair, ease of washing out after applying onto hair, touch and had a characteristic of soft touch.

| Formulation 4 : Hair dye (one-component type) | |
|---|---|
| Xyloglucan | 6.0 |
| Naphthol Blue Black (D & C Black No.1) | 0.2 |
| Alizurol Purple (EXT. D & C Violet No.2) | 0.3 |
| Sunset Yellow FCF (FD & C Yellow No.6) | 0.1 |
| Benzyl alcohol | 5.0 |
| Aluminum chloride hexahydrate | 0.2 |
| Methylphenylpolysiloxane | 0.5 |
| (This is a methylphenylpolysiloxane of the above-mentioned general formula (2). In the formula 1=5 to 20.) | |
| Tetrahydrofurfuryl alcohol | 12.0 |
| Glycerine | 0.5 |
| Polyoxyethylene (100) hydrogenated castor oil | 1.0 |
| Hydrolyzed keratin quaternary salts | 0.1 |
| Lactic acid | 2.0 |
| Perfume | Suitable amount |
| Ion-exchange water | Balance |

### <Production method>

The production method was in accordance with a standard method.

The hair dye thus obtained exhibited excellent dyeing ability, color-sustaining ability, handling performance and stability. It also exhibited uniform dyeing, no dropping from hair, ease of washing out after applying onto hair, touch and had a characteristic of soft touch.

| Formulation 5 : Hair dye (one-component type) | |
|---|---|
| Xyloglucan | 7.0 |
| Naphthol Blue Black (D & C Black No.1) | 0.2 |
| Alizurol Purple (EXT. D & C Violet No.2) | 0.3 |
| Sunset Yellow FCF (FD & C Yellow No.6) | 0.1 |
| Benzyl alcohol | 5.0 |
| Magnesium Chloride dihydrate | 1.0 |
| Polyether-modified polysiloxane | 0.5 |
| (This is a polyether-modified polysiloxane of the above-mentioned general formula (4). In the formula R¹ is hydrogen, o=50 to 60, p=2 to 5, q=8 to 10, r=0) | |
| Dipropylene glycol | 12.0 |
| 1,3-butylene glycol | 0.5 |
| Polyoxyethylene (100) hydrogenated castor oil | 1.0 |
| Sodium chondroitin sulfate | 0.1 |
| Bridged sodium polyacrylate | 0.2 |
| Hydrolyzed elastin | 0.1 |
| Lactic acid | 2.0 |
| Perfume | Suitable amount |
| Ion-exchange water | Balance |

### <Production method>

The production method was in accordance with a normal production method.

The hair dye thus obtained exhibited excellent dyeing ability, color-sustaining ability, handling performance and stability. It also exhibited uniform dyeing, no dropping from hair, ease of washing out after applying onto hair, touch and had a characteristic of soft touch.

| Formulation 6 : Color rinse (one-component type) | |
|---|---|
| Xyloglucan | 1.0 |
| Xanthanegum | 0.5 |
| Naphthol Blue Black (D & C Black No.1) | 0.2 |
| Alizurol Purple (EXT. D & C Violet No.2) | 0.3 |
| Sunset Yellow FCF (FD & C Yellow No.6) | 0.1 |
| Benzyl alcohol | 3.0 |
| Potassium chloride dihydrate | 0.3 |
| Polyether-modified polysiloxane | 0.5 |
| (This is a polyether-modified polysiloxane of the above-mentioned general formula (4). In the formula R¹ is hydrogen, o=50 to 60, p=2 to 5, q=8 to 10, r=0) | |
| Dimethylpolysiloxane | 0.5 |
| (This is a dimethylpolysiloxane of the above-mentioned general formula (13). In the formula R⁸ and R⁹ is methyl group and c=10000.) | |
| Octamethyltetrasiloxane | 3.0 |
| Cetanol | 8.0 |
| Glycerine | 0.5 |
| 1,3-butylene glycol | 15.0 |
| Polyoxyethylene (100) hydrogenated castor oil | 0.7 |
| hydrolyzed silk protein quaternary salt | 0.2 |
| Lactic acid | 0.6 |
| Perfume | Suitable amount |
| Ion-exchange water | Balance |

### <Production method>

The production method was in accordance with a standard method.

The color rinse thus obtained exhibited excellent dyeing ability, color-sustaining ability, handling performance and stability. It also exhibited uniform dyeing, no dropping from hair, ease of washing out after applying onto hair, touch and had a characteristic of soft touch.

| Formulation 7 : Hair dye (one-component type) | |
|---|---|
| Xyloglucan | 3.0 |
| Carboxyvinylpolymer | 3.0 |
| Naphthol Blue Black (D & C Black No.1) | 0.2 |
| Alizurol Purple (EXT. D & C Violet No.2) | 0.1 |
| Sunset Yellow FCF (FD & C Yellow No.6) | 0.2 |
| Benzyl alcohol | 5.0 |
| Calcium chloride dihydrate | 1.0 |
| Polyether-modified polysiloxane | 0.5 |
| (This is a polyether-modified polysiloxane of the above-mentioned general formula (4). In the formula R¹ is hydrogen, o=50 to 60, p=2 to 5, q=8 to 10, r=0) | |
| 1,3-butylene glycol | 15.0 |
| hydrolyzed keratin quaternary salt | 0.1 |
| Lactic acid | 2.0 |
| Perfume | Suitable amount |
| Ion-exchange water | Balance |

### <Production method>

The production method was in accordance with a standard method.

The hair dye thus obtained exhibited excellent dyeing ability, color-sustaining ability, handling performance and stability. It also exhibited uniform dyeing, no dropping from hair, ease of washing out after applying onto hair, touch and had a characteristic of soft touch.

| Formulation 8 : Hair dye (one-component type) | |
|---|---|
| Xyloglucan | 5.0 |
| Naphthol Blue Black (D & C Black No.1) | 0.2 |
| Alizurol Purple (EXT. D & C Violet No.2) | 0.3 |
| Sunset Yellow FCF (FD & C Yellow No.6) | 0.1 |
| Benzyl alcohol | 5.0 |
| Aluminum chloride hexahydrate | 1.0 |
| Amino-modified polysiloxane | 0.5 |
| (This is a Amino-modified polysiloxane of the above-mentioned general formula (6). In the formula R² is hydroxyl group, s=100, t=100) | |
| Tetrahydrofurfuryl alcohol | 12.0 |
| Polyoxyethylene (100) hydrogenated castor oil | 1.0 |
| Sodium pyrrolidone carboxylate | 0.5 |
| Hydrolyzed silk protein | 0.1 |
| Lactic acid | 2.0 |
| Perfume | Suitable amount |
| Ion-exchange water | Balance |

### <Production method>

The production method was in accordance with a standard method.

The hair dye thus obtained exhibited excellent dyeing ability, color-sustaining ability, handling performance and stability. It also exhibited uniform dyeing, no dropping from hair, ease of washing out after applying onto hair, touch and had a characteristic of soft touch.

| Formulation 9 : Hair dye (one-component type) | |
|---|---|
| Xyloglucan | 4.0 |
| Naphthol Blue Black (D & C Black No.1) | 0.2 |
| Alizurol Purple (EXT. D & C Violet No.2) | 0.3 |
| Sunset Yellow FCF (FD & C Yellow No.6) | 0.1 |
| Benzyl alcohol | 5.0 |
| Aluminum chloride hexahydrate | 1.0 |
| Dimethylpolysiloxane | 0.5 |
| (This is a dimethylpolysiloxane of the above-mentioned general formula (13). In the formula R⁸ and R⁹ are methyl group, c=10000) | |
| Tetrahydrofurfuryl alcohol | 12.0 |
| Propylene glycol | 5.0 |
| Polyoxyethylene (100) hydrogenated castor oil | 1.0 |
| Hydrolyzed keratin | 0.1 |
| Lactic acid | 2.0 |
| Perfume | Suitable amount |
| Ion-exchange water | Balance |

### <Production method>

The production method was in accordance with a standard method.

The hair dye thus obtained exhibited excellent dyeing ability, color-sustaining ability, handling performance and stability. It also exhibited uniform dyeing, no dropping from hair, ease of washing out after applying onto hair, touch and had a characteristic of soft touch.

| Formulation 10 : Hair dye (one-component type) | |
|---|---|
| Xyloglucan | 7.0 |
| Carboxymethyl cellulose | 0.1 |
| Naphthol Blue Black (D & C Black No.1) | 0.2 |
| Alizurol Purple (EXT. D & C Violet No.2) | 0.3 |
| Sunset Yellow FCF (FD & C Yellow No.6) | 0.1 |
| Benzyl alcohol | 5.0 |
| Aluminum chloride hexahydrate | 0.5 |
| Polyether-modified polysiloxane | 0.5 |
| (This is a polyether-modified polysiloxane of the above-mentioned general formula (4). In the formula R¹ is hydrogen, o=50 to 60, p=2 to 5, q=8 to 10, r=0) | |
| Tetrahydroforfuryl alcohol | 12.0 |
| Glycerine | 0.5 |
| Polyoxyethylene (100) hydrogenated castor oil | 1.0 |
| Hydrolyzed keratin quaternary salt | 0.2 |
| Lactic acid | 2.0 |
| Perfume | Suitable amount |
| Ion-exchange water | Balance |

### <Production method>

The production method was in accordance with a standard method.

The hair dye thus obtained exhibited excellent dyeing ability, color-sustaining ability, handling performance and stability. It also exhibited uniform dyeing, no dropping from hair, ease of washing out after applying onto hair, touch and had a characteristic of soft touch.

| Formulation 11 : Hair dye (one-component type) | |
|---|---|
| Xyloglucan | 1.5 |
| Xanthangum | 0.5 |
| Naphthol Blue Black (D & C Black No.1) | 0.02 |
| Alizurol Purple (EXT. D & C Violet No.2) | 0.03 |
| Sunset Yellow FCF (FD & C Yellow No.6) | 0.01 |
| Benzyl alcohol | 3.0 |
| Aluminum chloride hexahydrate | 0.6 |
| Polyether-modified polysiloxane | 0.2 |
| (This is a polyether-modified polysiloxane of the above-mentioned general formula (4). In the formula R¹ is hydrogen, o=50 to 60, p=2 to 5, q=8 to 10, r=0) | |
| Amino-modified polysiloxane | 0.5 |
| (This is a amino-modified polysiloxane of the above-mentioned general formula (5). In the formula R² is hydroxyl group, o=50 to 60, p=2 to 5, q=8 to 10, r=0) | |
| Octamethylcyclotetrasiloxane | 3.0 |
| Cetanol | 3.0 |
| Glycerine | 0.5 |
| 1,3-butylene glycol | 15.0 |
| Polyoxyethylene (100) hydrogenated castor oil | 0.7 |
| Stearyltrimethylammonium chloride | 0.1 |
| Hydrolyzed keratin | 0.2 |
| Lactic acid | 2.0 |
| Perfume | Suitable amount |
| Ion-exchange water | Balance |

### <Production method>

The production method was in accordance with a standard method.

The hair dye thus obtained exhibited excellent dyeing ability, color-sustaining ability, handling performance and stability. It also exhibited uniform dyeing, no dropping from hair, ease of washing out after applying onto hair, touch and had a characteristic of soft touch.

| Formulation 12 : Hair dye (one-component type) | |
|---|---|
| Xyloglucan | 3.0 |
| Naphthol Blue Black (D & C Black No.1) | 0.02 |
| Alizurol Purple (EXT. D & C Violet No.2) | 0.03 |
| Sunset Yellow FCF (FD & C Yellow No.6) | 0.01 |
| Benzyl alcohol | 3.0 |
| Aluminum chloride hexahydrate | 0.6 |
| Amino-modified polysiloxane | 0.6 |
| (This is a amino-modified polysiloxane of the above-mentioned general formula (5). In the formula R² is hydroxyl group, s=100, t=100) | |
| Octamethylcyclotetrasiloxane | 3.0 |
| Cetanol | 1.0 |
| Glycerine | 0.5 |
| Polyoxyethylene (100) hydrogenated castor oil | 0.7 |
| Hydrolyzed soy peptide | 0.2 |
| Lactic acid | 0.6 |
| Perfume | Suitable amount |
| Ion-exchange water | Balance |

### <Production method>

The production method was in accordance with a standard method.

The hair dye thus obtained exhibited excellent dyeing ability, color-sustaining ability, handling performance and stability. It also exhibited uniform dyeing, no dropping from hair, ease of washing out after applying onto hair, touch and had a characteristic of soft touch.

| Formulation 13 : Hair dye (one-component type) | |
|---|---|
| Xyloglucan | 3.0 |
| Naphthol Blue Black (D & C Black No.1) | 0.2 |
| Alizurol Purple (EXT. D & C Violet No.2) | 0.3 |
| Sunset Yellow FCF (FD & C Yellow No.6) | 0.1 |
| Benzyl alcohol | 5.0 |
| Magnesium chloride dihydrate | 1.0 |
| Dimethylpolysiloxane | 0.5 |
| (This is a silicone polymer of the above-mentioned general formula (13). In the formula R⁸ and R⁹ are methyl group, c=10000.) | |
| Glycerine | 0.5 |
| N-methyl-2-pyrrolidone | 12.0 |
| Polyoxyethylene (100) hydrogenated castor oil | 1.0 |
| Hydrolyzed collagen quaternary salt | 0.1 |
| Perfume | Suitable amount |
| Ion-exchange water | Balance |

### <Production method>

The production method was in accordance with a standaard method.

The hair dye thus obtained exhibited excellent dyeing ability, color-sustaining ability, handling performance and stability. It also exhibited uniform dyeing, no dropping from hair, ease of washing out after applying onto hair, touch and had a characteristic of soft touch.

| Formulation 14 : Hair dye (one-component type) | |
|---|---|
| Xyloglucan | 2.0 |
| Naphthol Blue Black (D & C Black No.1) | 0.2 |
| Alizurol Purple (EXT. D & C Violet No.2) | 0.3 |
| Sunset Yellow FCF (FD & C Yellow No.6) | 0.1 |
| Benzyl alcohol | 5.0 |
| Aluminum chloride hexahydrate | 1.0 |
| Polyether-modified polysiloxane | 0.5 |
| (This is a polyether-modified polysiloxane of the above-mentioned general formula (4). In the formula R¹ is hydrogen, o=5 to 10, p=4 to 6, q=9 to 13, r=0) | |
| 1,3-butylene glycol | 12.0 |
| Octamethyltetrasiloxane | 3.0 |
| Glycerine | 0.5 |
| Polyoxyethylene hydrogenated castor oil | 1.0 |
| Bridged sodium polyacrylate | 0.2 |
| Hydrolyzed soy peptide quaternary salt | 0.2 |
| Perfume | Suitable amount |
| Ion-exchange water | Balance |

### <Production method>

The production method was in accordance with a standard method.

The hair dye thus obtained exhibited excellent dyeing ability, color-sustaining ability, handling performance and stability. It also exhibited uniform dyeing, no dropping from hair, ease of washing out after applying onto hair, touch and had a characteristic of soft touch.

| Formulation 15 : Color rinse | |
|---|---|
| Xyloglucan | 2.0 |
| Naphthol Blue Black (D & C Black No.1) | 0.2 |
| Alizurol Purple (EXT. D & C Violet No.2) | 0.3 |
| Sunset Yellow FCF (FD & C Yellow No.6) | 0.1 |
| Benzyl alcohol | 3.0 |
| Aluminum chloride hexahydrate | 0.8 |
| Polyether-modified polysiloxane | 0.5 |
| (This is a polyether-modified polysiloxane of the above-mentioned general formula (4). In the formula R¹ is hydrogen, o=50 to 60, p=2 to 5, q=8 to 10, r=0) | |
| Octamethylcyclotetrasiloxane | 3.0 |
| Tetrahydrofurfuryl alcohol | 8.0 |
| Glycerine | 0.5 |
| 1,3-butylene glycol | 15.0 |
| Polyoxyethylene (100) hydrogenated castor oil | 0.7 |
| Hydrolyzed collagen quaternary salt | 0.2 |
| Perfume | Suitable amount |
| Ion-exchange water | Balance |

### <Production method>

The production method was in accordance with a standard method.

The color rinse thus obtained exhibited excellent dyeing ability, color-sustaining ability, handling performance and stability. It also exhibited uniform dyeing, no dropping from hair, ease of washing out after applying onto hair, touch and had a characteristic of soft touch.

| Formulation 16 : Hair dye | |
|---|---|
| Xyloglucan | 5.0 |
| Naphthol Blue Black (D & C Black No.1) | 0.2 |
| Alizurol Purple (EXT. D & C Violet No.2) | 0.3 |
| Sunset Yellow FCF (FD & C Yellow No.6) | 0.1 |
| Benzyl alcohol | 5.0 |
| Calcium chloride dihydrate | 0.6 |
| Octylpolyglycoside | 1.0 |
| Tetrahydrofurfuryl alcohol | 12.0 |
| Hydrolyzed keratin | 0.1 |
| Lactic acid | 2.0 |
| Perfume | Suitable amount |
| Ion-exchange water | Balance |

### <Production method>

The production method was in accordance with a standard method.

The hair dye thus obtained exhibited excellent dyeing ability, color-sustaining ability, handling performance and stability. It also exhibited uniform dyeing, no dropping from hair, ease of washing out after applying onto hair, touch and had a characteristic of soft touch.

| Formulation 17 : Color rinse (one-component type) | |
|---|---|
| Xyloglucan | 0.5 |
| Hydroxyethyl cellulose | 1 |
| Naphthol Blue Black (D & C Black No.1) | 0.01 |
| Orange II (D & C Orange No.4) | 0.01 |
| Alizurol Purple (EXT. D & C Violet No.2) | 0.01 |
| Fast Acid Magenta (D & C Red No.33) | 0.01 |
| Benzyl alcohol | 8 |
| Aluminum chloride hexahydrate | 0.5 |
| Amino-modified silicone | 0.5 |
| 1,3-butylene glycol | 15 |
| Cetanol | 1 |
| Polyoxyethylene (100) hydrogenated castor oil | 0.5 |
| α-Olefinsulfonic acid | 0.5 |
| Ion-exchange water | Balance |

### <Production method>

The production method was in accordance with a standard method.

The color rinse thus obtained exhibited excellent dyeing ability, color-sustaining ability, handling performance and stability. It also exhibited uniform dyeing, no dropping from hair, ease of washing out after applying onto hair, touch and had a characteristic of soft touch.

| Formulation 18 : Hair dye (one-component type) | |
|---|---|
| Xyloglucan | 3.0 |
| Xanthan gum | 0.5 |
| Naphthol Blue Black (D & C Black No.1) | 0.01 |
| Alizurol Purple (EXT. D & C Violet No.2) | 0.01 |
| Tartrazine (FD & C Yellow No.5) | 0.1 |
| Benzyl alcohol | 5.0 |
| Aluminum chloride hexahydrate | 0.6 |
| Tetrahydrofurfuryl alcohol | 12.0 |
| Hydrolyzed collagen | 0.2 |
| Perfume | Suitable amount |
| Ion-exchange water | Balance |

### <Production method>

The production method was in accordance with a standard method.

The hair dye thus obtained exhibited excellent dyeing ability, color-sustaining ability, handling performance and stability. It also exhibited uniform dyeing, no dropping from hair, ease of washing out after applying onto hair, touch and had a characteristic of soft touch.

| Formulation 19 : Hair dye (one-component type) | |
|---|---|
| Xyloglucan | 4.0 |
| Naphthol Blue Black (D & C Black No.1) | 0.1 |
| Alizurol Purple (EXT. D & C Violet No.2) | 0.2 |
| Tartrazine (FD & C Yellow No.5) | 0.4 |
| Benzyl alcohol | 5.0 |
| Aluminum chloride hexahydrate | 1.0 |
| Dipropylene glycol | 10.0 |
| Carboxyvinylpolymer | 2.0 |
| Hydrolyzed keratin quaternary salt | 0.2 |
| Perfume | Suitable amount |
| Ion-exchange water | Balance |

### <Production method>

The production method was in accordance with a standard method.

The hair dye thus obtained exhibited excellent dyeing ability, color-sustaining ability, handling performance and stability. It also exhibited uniform dyeing, no dropping from hair, ease of washing out after applying onto hair, touch and had a characteristic of soft touch.

| Formulation 20 : Color rinse (one-component type) | |
|---|---|
| Xyloglucan | 2 |
| Naphthol Blue Black (D & C Black No.1) | 0.01 |
| Orange II (D & C Orange No.4) | 0.01 |
| Alizurol Purple (EXT. D & C Violet No.2) | 0.01 |
| Fast Acid Magenta (D & C Red No.33) | 0.01 |
| Benzyl alcohol | 8 |
| Aluminum chloride hexahydrate | 0.5 |
| 1,3-butylene glycol | 15 |
| Cetanol | 1 |
| α -Olefinsulfonic acid | 0.5 |
| Polyoxyethylene hydrogenated castor oil | 0.5 |
| Amino-modified silicone | 0.5 |
| Lactic acid | 0.1 |
| Sodium lactate | 0.1 |
| Ion-exchange water | Balance |

### <Production method>

The production method was in accordance with a production method of a color rinse of Formulation 1.

The color rinse thus obtained exhibited excellent dyeing ability, color-sustaining ability, handling performance and stability. It also exhibited uniform dyeing, no dropping from hair, ease of washing out after applying onto hair, touch and had a characteristic of soft touch.

| Formulation 21 : Hair dye (one-component type) | |
|---|---|
| Xyloglucan | 2 |
| Naphthol Blue Black (D & C Black No.1) | 0.01 |
| Orange II (D & C Orange No.4) | 0.01 |
| Alizurol Purple (EXT. D & C Violet No.2) | 0.01 |
| Fast Acid Magenta (D & C Red No.33) | 0.01 |
| Benzyl alcohol | 8 |
| Aluminum chloride hexahydrate | 0.1 |
| 1,3-butylene glycol | 15 |
| Cetanol | 1 |
| α -Olefinsulfonic acid | 0.5 |
| Polyoxyethylene hydrogenated castor oil | 0.5 |
| Amino-modified silicone | 0.5 |
| Lactic acid | 1 |
| Sodium lactate | 1 |
| Ion-exchange water | Balance |

### <Production method>

The production method was in accordance with a production method of a color rinse of Formulation 1.

The color rinse thus obtained exhibited excellent dyeing ability, color-sustaining ability, handling performance and stability. It also exhibited uniform dyeing, no dropping from hair, ease of washing out after applying onto hair, touch and had a characteristic of soft touch.

| Formulation 22 : Hair dye (one-component type) | |
|---|---|
| Xyloglucan | 2 |
| Naphthol Blue Black (D & C Black No.1) | 0.01 |
| Orange II (D & C Orange No.4) | 0.01 |
| Alizurol Purple (EXT. D & C Violet No.2) | 0.01 |
| Fast Acid Magenta (D & C Red No.33) | 0.01 |
| Benzyl alcohol | 8 |
| Aluminum chloride hexahydrate | 0.5 |
| 1,3-butylene glycol | 15 |
| Cetanol | 1 |
| α -Olefinsulfonic acid | 0.5 |
| Polyoxyethylene hydrogenated castor oil | 0.5 |
| Amino-modified silicone | 0.5 |
| Lactic acid | 1 |
| Sodium lactate | 1 |
| Ion-exchange water | Balance |

### <Production method>

The production method was in accordance with a production method of a color rinse of Formulation 1.

The color rinse thus obtained exhibited excellent dyeing ability, color-sustaining ability, handling performance and stability. It also exhibited uniform dyeing, no dropping from hair, ease of washing out after applying onto hair, touch and had a characteristic of soft touch.

| Formulation 23 : Hair dye (one-component type) | |
|---|---|
| Xyloglucan | 1.0 |
| Agar | 2.0 |
| Orange II (D & C Orange No.4) | 0.4 |
| Benzyl alcohol | 8.0 |
| Aluminum chloride hexahydrate | 1.0 |
| Ethanol | 20.0 |
| Glycolic acid | 1.5 |
| Sodium lactate | 0.3 |
| Ion-exchange water | Balance |

### <Production method>

The production method was in accordance with a standard method.

The hair dye thus obtained exhibited excellent dyeing ability, handling performance, color-sustaining ability and stability.

| Formulation 24 : Hair dye (A mixing type hair dye wherein a mixture of two components is prepared before use and the mixture is applied to hair) | |
|---|---|
| First Component | |
| Ethanol | 12.0 |
| Aluminum chloride hexahydrate | 0.5 |
| Glycollic acid | 2.0 |
| Ion-exchange water | to 40 |

| Second Component | |
|---|---|
| Hydroxyethyl cellulose | 1.0 |
| Agar | 2.0 |
| Orange II (D & C Orange No.4) | 0.4 |
| Acid red | 0.1 |
| Benzyl alcohol | 4.0 |
| Ethanol | 8.0 |
| Sodium lactate | 0.5 |
| Ion-exchange water | to 60.0 |

### <Production method>

The production method was in accordance with a standard method.

The hair dye thus obtained exhibited excellent dyeing ability, color-sustaining ability, handling performance and stability.

| Formulation 25 : Hair dye (A mixing type hair dye wherein a mixture of two component is prepared before use and the mixture is applied to hair) | |
|---|---|
| First Component | |
| Ethanol | 20.0 |
| Aluminum chloride hexahydrate | 1.0 |
| Benzyl alcohol | 8.0 |
| Glycolic acid | 1.0 |
| An acid dye | Suitable amount |
| Ion-exchange water | to 40.0 |

| Second Component | |
|---|---|
| Hydroxyethyl cellulose | 1.0 |
| Agar | 2.0 |
| Ethanol | 4.0 |
| Methyl paraben | 0.1 |
| Ion-exchange water | to 60.0 |

### <Production method>

The production method was in accordance with a standard method.

The hair dye thus obtained exhibited excellent dyeing ability, color-sustaining ability, handling performance and stability.

| Formulation 26 : Hair dye (A mixing type hair dye wherein a mixture of two components is prepared before use and the mixture is applied to hair) | |
|---|---|
| First Component | |
| Ethanol | 12.0 |
| Aluminum chloride hexahydrate | 0.5 |
| Glycolic acid | 2.0 |
| Orange II (D & C Orange No.4) | 0.001 |
| Ion-exchange water | to 40.0 |

| Second Component | |
|---|---|
| Xyloglucan | 1.0 |
| Hydroxyethyl cellulose | 0.5 |
| Orange II (D & C Orange No.4) | 0.4 |
| Acid red | 0.1 |
| Benzyl alcohol | 4.0 |
| Ethanol | 8.0 |
| Sodium lactate | 0.5 |
| Ion-exchange water | to 60.0 |

### <Production method>

The production method was in accordance with a standard method.

The hair dye thus obtained exhibited excellent dyeing ability, color-sustaining ability, handling performance and stability.

| Formulation 27 : Hair dye fixative | |
|---|---|
| Aluminum chloride hexahydrate | 1.0 |
| Glycolic acid | 1.0 |
| Ethanol | 20.0 |
| Benzyl alcohol | 8.0 |
| Dipropylene glycol | 15.0 |
| Ion-exchange water | balance |

### <Production method>

The production method was in accordance with a standard method.

The hair dye thus obtained exhibited excellent dyeing ability, color-sustaining ability, handling performance and stability.

| Formulation 28 : Hair dye fixative (Mist type) | |
|---|---|
| Aluminum chloride hexahydrate | 1.0 |
| Glycolic acid | 1.0 |
| Ethanol | 20.0 |
| Benzyl alcohol | 8.0 |
| Dipropylene glycol | 15.0 |
| Ion-exchange water | balance |

### <Production method>

The production method was in accordance with a standard method.

The hair dye thus obtained exhibited excellent dyeing ability, color-sustaining ability, handling performance and stability.

| Formulation 29 : hair dye fixative (Treatment type) | |
|---|---|
| Aluminum chloride hexahydrate | 3 |
| Isostearyl alcohol | 0.5 |
| Octyl palmitate | 2 |
| Stearyl alcohol | 5 |
| Stearyl trimethyl ammonium chloride | 1 |
| Glycolic acid | 3 |
| Sodium lactate | 1.5 |
| Benzyl alcohol | 2 |
| Dipropylene glycol | 3 |
| Perfume | 0.3 |
| Ion-exchange water | balance |

### <Production method>

Aluminum chloride hexahydrate, glycollic acid, Sodium hydrate are dissolved into ion-exchange water followed by dissolving stearyl trimethyl ammonium chloride into the mixture at 70°C. Then benzyl alcohol and dipropylene glycol are added into the mixture with mixing, followed by adding isostearyl alcohol, octyl palmitate and stearyl alcohol into the mixture. After dissolution of these ingredients is confirmed the mixture is treated by a homomixer. Then the mixture is cooled to less than 50°C and then perfume is added into the mixture.

The hair dye thus obtained exhibited excellent dyeing ability, color-sustaining ability, handling performance and stability.

| Formulation 30 : Hair dye (A mixing type hair dye wherein a mixture of two components is prepared before use and the mixture is applied to hair) | |
|---|---|
| First Component | |
| Aluminum chloride hexahydrate | 1 |
| Isostearyl alcohol | 0.5 |
| Stearyl alcohol | 2 |
| Stearyl trimethyl ammonium chloride | 1 |
| Glycollic acid | 1.5 |
| Sodium lactate | 0.7 |
| Benzyl alcohol | 2 |
| Ethanol | 2 |
| Dipropylene glycol | 2 |
| Perfume | 0.5 |
| Ion-exchange water | to 40.0 |

| Second Component | |
|---|---|
| Carboxyvinylpolymer | 3.0 |
| Orange II (D & C Orange No.4) | 0.4 |
| New Coccin | 0.1 |
| Benzyl alcohol | 8.0 |
| Dipropylene glycol | 15.0 |
| Sodium hydroxide | 0.03 |
| Ion-exchange water | to 60.0 |

### <Production method>

The production method of First Component : Aluminum chloride hexahydrate, glycollic acid, Sodium hydrate are dissolved into ion-exchange water followed by dissolving stearyl trimethyl ammonium chloride into the mixture at 70°C. Then benzyl alcohol and dipropylene glycol are added into the mixture with mixing, followed by adding isostearyl alcohol and stearyl alcohol into the mixture. After dissolution of these ingredients is confirmed the mixture is treated by a homomixer. Then the mixture is cooled to less than 50°C and then ethanol and perfume is added into the mixture.

The production method of Second Component was in accordance with a standard method.

The hair dye thus obtained exhibited excellent dyeing ability, color-sustaining ability, handling performance and stability.

| Formulation 31 : Color rinse (one-component type) | |
|---|---|
| Hydroxyethyl cellulose | 2.0 |
| Benzyl alcohol | 8.0 |
| Naphthol Blue Black (D & C Black No.1) | 0.01 |
| Orange II (D & C Orange No.4) | 0.01 |
| Alizurol Purple (EXT. D & C Violet No.2) | 0.01 |
| Fast Acid Magenta (D & C Red No.33) | 0.01 |
| Ethanol | 15 |
| 1,3-Butylene glycol | 5 |
| Aluminum Chloride hexahydrate | 0.2 |
| Cetanol | 1 |
| α-Olefinsulfonic acid | 0.5 |
| Polyoxyethylene hydrogenated castor oil | 0.5 |
| Stearyl trimethyl ammonium chloride | 0.1 |
| Amino-modified silicone | 0.5 |
| Ion-exchange water | Balance |

### <Production method>

Cetanol is added to 1,3-butylene glycol and dissolved with heating at 70°C and then combined with α-olefinsulfonic acid and polyoxyethylene hydrogenated castor oil (organic phase). Separately, aluminum chloride is dissolved in an ion-exchange water followed by dissolution of hydroxypropyl cellulose at 70°C (aqueous phase). To the organic phase, the aqueous phase is added followed by benzyl alcohol, ethanol and finally a dye dissolved in an ion-exchange water, and mixed.

The color rinse thus obtained exhibited excellent dyeing ability, color-sustaining ability, handling performance and stability.

| Formulation 32 : Color rinse (one-component type) | |
|---|---|
| Hydroxyethyl cellulose | 2 |
| Benzyl alcohol | 8 |
| Naphthol Blue Black (D & C Black No.1) | 0.01 |
| Orange II (D & C Orange No.4) | 0.01 |
| Alizurol Purple (EXT. D & C Violet No.2) | 0.01 |
| Fast Acid Magenta (D & C Red No.33) | 0.01 |
| Ethanol | 20 |
| Aluminum Chloride hexahydrate | 0.4 |
| Cetanol | 1 |
| α-Olefinsulfonic acid | 0.5 |
| Polyoxyethylene hydrogenated castor oil | 0.5 |
| Stearyl trimethyl ammonium chloride | 0.1 |
| Amino-modified silicone | 0.5 |
| Ion-exchange water | Balance |

### <Production method>

The production method was in accordance with Formulation 31

The color rinse thus obtained exhibited excellent dyeing ability, color-sustaining ability, handling performance and stability.

| Formulation 33 : Color rinse (one-component type) | |
|---|---|
| Hydroxyethyl cellulose | 2 |
| Benzyl alcohol | 5 |
| Naphthol Blue Black (D & C Black No.1) | 0.01 |
| Orange II (D & C Orange No.4) | 0.02 |
| Alizurol Purple (EXT. D & C Violet No.2) | 0.01 |
| Fast Acid Magenta (D & C Red No.33) | 0.01 |
| Ethanol | 10 |
| Aluminum Chloride hexahydrate | 0.5 |
| Cetanol | 3 |
| α-Olefinsulfonic acid | 0.5 |
| Polyoxyethylene hydrogenated castor oil | 0.5 |
| Dimethylpolysiloxane | 2 |
| (This is a dimethylpolysiloxane of the above-mentioned general formula (1). | |
| In the formula k=25) | |
| Stearyl trimethyl ammonium chloride | 0.1 |
| Amino-modified silicone | 0.5 |
| (This is an amino-modified polysiloxane of the above-mentioned general formula (6). In the formula s=10000, t=10.) | |
| Ion-exchange water | Balance |

### <Production method>

The production method was in accordance with Formulation 31

The color rinse thus obtained exhibited excellent dyeing ability, color-sustaining ability, handling performance and stability.

| Formulation 34 : Color rinse (one-component type) | |
|---|---|
| Hydroxyethyl cellulose | 1.5 |
| Benzyl alcohol | 3 |
| Naphthol Blue Black (D & C Black No.1) | 0.03 |
| Orange II (D & C Orange No.4) | 0.03 |
| Alizurol Purple (EXT. D & C Violet No.2) | 0.02 |
| Fast Acid Magenta (D & C Red No.33) | 0.01 |
| Ethanol | 5 |
| 1,3-butylene glycol | 15 |
| Aluminum Chloride hexahydrate | 0.8 |
| Cetanol | 1 |
| α-Olefinsulfonic acid | 0.5 |
| Silicone polymer | 1 |
| (This is a silicone polymer of the above-mentioned general formula (13). | |
| In the formula c=8000.) | |
| Polyoxyethylene hydrogenated castor oil | 0.8 |
| Dimethylpolysiloxane | 4 |
| (This is a dimethylpolysiloxane of the above-mentioned general formula (1). In the formula k=25) | |
| Stearyl trimethyl ammonium chloride | 0.1 |
| Amino-modified silicone | 1 |
| (This is an amino-modified polysiloxane of the above-mentioned general formula (6). In the formula R² is methyl group and s=10000, t=100.) | |
| Ion-exchange water | Balance |

### <Production method>

The production method was in accordance with Formulation 31

The color rinse thus obtained exhibited excellent dyeing ability, color-sustaining ability, handling performance and stability.

| Formulation 35 : Color rinse (one-component type) | |
|---|---|
| Hydroxyethyl cellulose | 1.5 |
| Benzyl alcohol | 8 |
| Naphthol Blue Black (D & C Black No.1) | 0.03 |
| Orange II (D & C Orange No.4) | 0.08 |
| Alizurol Purple (EXT. D & C Violet No.2) | 0.05 |
| Fast Acid Magenta (D & C Red No.33) | 0.02 |
| Ethanol | 5 |
| 1,3-butylene glycol | 15 |
| Aluminum Chloride hexahydrate | 1 |
| Cetanol | 3 |
| α-Olefinsulfonic acid | 0.1 |
| Polyoxyethylene hydrogenated castor oil | 0.8 |
| Dimethylpolysiloxane | 2 |
| (This is a dimethylpolysiloxane of the above-mentioned general formula (1). In the formula k=20) | |
| Stearyl trimethyl ammonium chloride | 0.1 |
| Amino-modified silicone | 2.5 |
| (This is an amino-modified polysiloxane of the above-mentioned general formula (6). In the formula R² is methyl group and s=10000, t=100.) | |
| Ion-exchange water | Balance |

### <Production method>

The production method was in accordance with Formulation 31

The color rinse thus obtained exhibited excellent dyeing ability, color-sustaining ability, handling performance and stability.

As detailed above, a fixative, a hair dye and a hair dyeing method according to the invention improve the dyeing ability and the color-sustaining ability greatly since a complex nucleus is employed for fixing an acid dye to effect the complex formation of the acid dye within a hair which leads to an insolubility and a higher apparent molecular weight.

## Claims

**1.** A hair dye fixative comprising a complex nucleus capable of forming a complex with an acid dye.

**2.** A hair dye fixative according to Claim 1 wherein said fixative comprises an acid.

**3.** A hair dye fixative according to Claim 1 wherein said fixative comprises silicone.

**4.** A hair dye fixative according to Claim 1 wherein said complex nucleus is a multivalent metal ion.

**5.** A hair dye fixative according to Claim 4 wherein a combination amount of said multivalent metal ion is 0.1 to 20 % by weight in terms of a weight of the corresponding metal salt.

**6.** A hair dye fixative according to Claim 1 wherein said complex nucleus is aluminum ion.

**7.** A hair dye fixative according to Claim 6 wherein a combination amount of aluminum ion is 0.1 to 20 % by weight in terms of a weight of the corresponding metal salt.

**8.** A hair dye fixative according to anyone of Claims 5 or 7 wherein said fixative comprises an acid in an amount of 0.01 to 15 % by weight.

**9.** A hair dye fixative according to Claim 8 wherein said acid is an organic acid.

**10.** A hair dye fixative according to Claim 8 wherein said acid is -hydroxyl acid.

**11.** A hair dye fixative according to Claim 8 wherein said acid is glycolic acid.

**12.** A hair dye fixative according to anyone of Claims 5 or 7 wherein said fixative comprises one or more compounds selected from ethanol, isopropanol, n-propanol, n-butanol and isobutanol.

**13.** A hair dye fixative according to anyone of Claims 5 or 7 wherein said fixative comprises silicone.

**14.** A hair dye comprising:
an acid dye;
a complex nucleus capable of forming a complex with said acid dye; and,
an organic solvent having quantitative and qualitative characteristics which do not allow said acid dye to form a dye complex with said complex nucleus.

**15.** A hair dye according to Claim 14 wherein said hair dye comprises xyloglucan.

**16.** A hair dye according to Claim 15 wherein said hair dye comprises silicone.

**17.** A hair dye according to Claim 14 wherein said hair dye comprises an acid.

**18.** A hair dye according to Claim 14 wherein said complex nucleus is a multivalent metal ion.

**19.** A hair dye according to Claim 18 wherein a combination amount of said multivalent metal ion is 0.01 to 20 % by weight in terms of a weight of the corresponding metal salt.

**20.** A hair dye according to Claim 14 wherein said complex nucleus is aluminum ion.

**21.** A hair dye according to Claim 20 wherein a combination amount of aluminum ion is 0.01 to 20 % by weight in terms of a weight of the corresponding metal salt.

**22.** A hair dye according to anyone of Claims 19 or 21 wherein said hair dye comprises xyloglucan in an amount of 0.1 to 30 % by weight.

**23.** A hair dye according to Claim 22 wherein said hair dye comprises silicone.

**24.** A hair dye according to anyone of Claims 19 or 21 wherein said hair dye comprises an acid in an amount of 0.01 to 15 % by weight.

**25.** A hair dye according to Claim 24 wherein said acid is an organic acid.

**26.** A hair dye according to Claim 24 wherein said acid is -hydroxyl acid.

**27.** A hair dye according to Claim 24 wherein said acid is glycolic acid.

**28.** A hair dye according to anyone of Claims 19 or 21 wherein said hair dye comprises an aromatic alcohol.

**29.** A hair dye according to anyone of Claims 19 or 21 wherein said hair dye comprises one or more compounds selected from ethanol, isopropanol, n-propanol, n-butanol and isobutanol.

**30.** A mixing type hair dye wherein a mixture of two compositions is prepared before use and the mixture is applied to hair consisting of:
a first composition comprising an acid dye; and,
a second composition comprising a complex nucleus capable of forming a complex with said acid dye.

**31.** A hair dye according to Claim 30 wherein a first composition comprises xyloglucan.

**32.** A hair dye according to Claim 31 wherein a first composition comprises silicone.

**33.** A hair dye according to Claim 30 wherein a second composition comprises an acid.

**33.** A hair dye according to Claim 30 wherein a second composition comprises an acid.

**34.** A hair dye according to Claim 30 wherein a second composition comprises silicone.

**35.** A hair dye according to Claim 30 wherein said complex nucleus is a multivalent metal ion.

**36.** A hair dye according to Claim 35 wherein a second composition comprises said multivalent metal in an amount of 0.1 to 20 % by weight in terms of a weight of the corresponding metal salt.

**37.** A hair dye according to Claim 30 wherein said complex nucleus is aluminum ion.

**38.** A hair dye according to Claim 37 wherein a second composition comprises aluminum ion in an amount of 0.1 to 20 % by weight in terms of a weight of the corresponding metal salt.

**39.** A hair dye according to anyone of Claims 36 or 38 wherein a first composition comprises xyloglucan in an amount of 0.1 to 30 % by weight.

**40.** A hair dye according to Claim 39 wherein a first composition comprises silicone.

**41.** A hair dye according to anyone of Claims 36 or 38 wherein a second composition comprises an acid in an amount of 0.01 to 15 % by weight.

**42.** A hair dye according to Claim 41 wherein said acid is an organic acid.

**43.** A hair dye according to Claim 41 wherein said acid is -hydroxyl acid.

**44.** A hair dye according to Claim 41 wherein said acid is glycolic acid.

**45.** A hair dye according to anyone of Claims 36 or 38 wherein a first composition comprises an aromatic alcohol.

**46.** A hair dye according to anyone of Claims 36 or 38 wherein a first composition comprises one or more compounds selected from ethanol, isopropanol, n-propanol, n-butanol and isobutanol.

**47.** A hair dye according to anyone of Claims 36 or 38 wherein a second composition comprises benzyl alcohol.

**48.** A hair dye according to anyone of Claims 36 or 38 wherein a second composition comprises one or more compounds selected from ethanol, isopropanol, n-propanol, n-butanol and isobutanol.

**49.** A hair dyeing method **characterized by** treating the hair with a hair dye fixative comprising a complex nucleus capable of forming a complex with an acid dye, and then dying a hair with an acid dye.

**50.** A hair dyeing method **characterized by** dyeing a hair with an acid dye and simultaneously treating the hair with a hair dye fixative comprising a complex nucleus capable of forming a complex with said acid dye.

**51.** A hair dyeing method **characterized by** dying a hair with an acid dye, and then treating the hair with a hair dye fixative comprising a complex nucleus capable of forming a complex with said acid dye.

**52.** A hair dyeing method according to anyone of Claims 49, 50 or 51 wherein said complex nucleus is a metal ion.

**53.** A hair dyeing method according to anyone of Claims 49, 50 or 51 wherein said complex nucleus is aluminum ion.
